(19) Europäisches Patentamt
European Patent Office
Office européen des brevets

(11) Veröffentlichungsnummer: **0 000 574**
**B1**

(12) # EUROPÄISCHE PATENTSCHRIFT

(45) Veröffentlichungstag der Patentschrift
**04.08.82**

(21) Anmeldenummer **78100489.0**

(22) Anmeldetag: **24.07.78**

(51) Int. Cl.³ **C 07 D 235/24,** C 07 D 235/12, C 07 D 405/00, C 07 D 409/00, C 07 D 401/00, A 61 K 31/415, C 07 C 49/80, C 07 C 87/58, C 07 C 49/76, C 07 C 87/52

(54) **Benzimidazol-2-Derivate, Verfahren zu ihrer Herstellung und ihre Verwendung zur Herstellung von Arzneimitteln.**

(30) Priorität: **01.08.77 HU CI001761**
**27.02.78 CH 2094/78**

(43) Veröffentlichungstag der Anmeldung:
**07.02.79 Patentblatt 79/3**

(45) Bekanntmachung des Hinweises auf die Patenterteilung:
**04.08.82 Patentblatt 82/31**

(84) Benannte Vertragsstaaten:
**BE CH DE FR GB LU NL SE**

(56) Entgegenhaltungen:
**CH-A-356 460**
**CH-A-356 461**
**CH-A-362 411**

(73) Patentinhaber: **CIBA-GEIGY AG, Patentabteilung Postfach, CH-4002 Basel (CH)**

(72) Erfinder: **Habicht, Ernst, Dr., Bienenstrasse 13, CH-4104 Oberwil (CH)**
Erfinder: **Ferrini, Pier Giorgio, Dr., Im Rehwechsel 22, CH-4102 Binningen (CH)**
Erfinder: **Sallmann, Alfred, Dr., Joachimackerstrasse 12, CH-4103 Bottmingen (CH)**

**EP 0 000 574 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Benzimidazol-2-Derivate, Verfahren zu ihrer Herstellung und ihre Verwendung zur Herstellung von Arzneimitteln

Die Erfindung betrifft substituierte Heterocyclylverbindungen, insbesondere benz-substituierte Benzimidazol-2-derivate der Formel

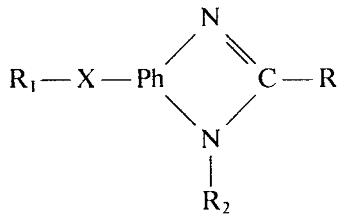

(I)

worin R eine gegebenenfalls veresterte oder amidierte Carboxygruppe oder eine gegebenenfalls verätherte oder veresterte Hydroxylmethylgruppe ist, $R_1$ einen aliphatischen, cycloaliphatischen, aromatischen, araliphatischen, heterocyclischen oder heterocyclisch-aliphatischen Rest darstellt, $R_2$ für Wasserstoff oder einen aliphatischen Rest steht, und Ph eine den Rest $R_1-X$ enthaltende 1,2-Phenylengruppe darstellt, X Niederalkyliden oder eine direkte Bindung bedeutet, und pharmazeutisch verwendbare Salze von Verbindungen der Formel I mit salzbildenden Eigenschaften zur Anwendung in einem Verfahren zur prophylaktischen und/oder therapeutischen Behandlung des menschlichen oder tierischen Körpers, diese enthaltende pharmazeutische Präparate, ihre Verwendung sowie neue Verbindungen der Formel I und Salze von Verbindungen der Formel I mit salzbildenden Eigenschaften, mit der Maßgabe, daß R von Alkoxymethyl oder $R_1-X$ von Niederalkyl verschieden ist, wenn $R_2$ Wasserstoff, Alkyl oder Alkenyl bedeutet, daß weiterhin R von unsubstituiertem Carbamyl oder $R_1-X$ von Alkyl verschieden ist, wenn $R_2$ Wasserstoff oder Alkyl bedeutet, und daß schließlich $R_1-X-Ph$ von in 4- und/oder 5-Stellung durch Methyl substituiertem 1,2-Phenylen verschieden ist, wenn R Carboxy, Carbamyl oder Hydroxymethyl darstellt und $R_2$ Wasserstoff bedeutet, und Verfahren zu ihrer Herstellung.

Die Erfindung betrifft beispielsweise Verbindungen der Formel I, worin R, $R_1$, Ph und $R_2$ die angegebenen Bedeutungen haben und entweder X Methylen bedeutet, wobei $R_1$ mindestens 2 Kohlenstoffatome aufweist, wenn Ph ansonsten unsubstituiert ist, $R_2$ Äthyl bedeutet und R Acetoxymethyl darstellt, oder X eine direkte Bindung bedeutet, wobei $R_1$ mindestens 2 Kohlenstoffatome aufweist, wenn Ph ansonsten unsubstituiert ist, $R_1-X$ von Alkyl verschieden ist, wenn R unsubstituiertes Carbamyl und $R_2$ Wasserstoff oder Alkyl oder R Alkoxymethyl und $R_2$ Wasserstoff, Alkyl oder Alkenyl bedeutet, und $R_1-X-Ph-$ von in 4- und/oder 5-Stellung durch Methyl substituiertem 1,2-Phenylen verschieden ist, wenn R Carboxy oder Hydroxymethyl und $R_2$ Wasserstoff bedeutet, und Salze solcher Verbindungen mit salzbildenden Eigenschaften, Verfahren zu ihrer Herstellung, diese enthaltende pharmazeutische Präparate und ihre Verwendung als Arzneimittel.

Im Zusammenhang mit der vorliegenden Beschreibung enthalten mit »nieder« bezeichnete organische Reste und Verbindungen insbesondere bis und mit 7, vorzugsweise bis und mit 4 Kohlenstoffatome.

In verestertem Carboxy und veräthertem Hydroxymethyl R bedeutet die verätherte Hydroxygruppe beispielsweise eine durch einen aliphatischen oder araliphatischen Rest, wie einen gegebenenfalls substituierten aliphatischen oder araliphatischen Kohlenwasserstoffrest, verätherte Hydroxygruppe, z. B. entsprechendes Niederalkoxy oder Phenylniederalkoxy. Substituenten von Niederalkoxy sind u. a. Hydroxy, Niederalkoxy und/oder Diniederalkylamino, und solche von Phenylniederalkoxy, z. B. Niederalkyl, Niederalkoxy und/oder Halogen, wobei einer oder mehrere Substituenten vorhanden sein können.

In amidiertem Carboxy bedeutet die Aminogruppe beispielsweise gegebenenfalls durch Hydroxy monosubstituiertes, durch Niederalkyl mono- oder disubstituiertes oder durch Niederalkylen disubstituiertes Amino.

In verestertem Hydroxymethyl R bedeutet die veresterte Hydroxygruppe beispielsweise mit einer Carbonsäure, wie einer aliphatischen oder aromatischen Carbonsäure verestertes Hydroxy, z. B. entsprechendes Niederalkanoyloxy oder gegebenenfalls durch Niederalkyl, Niederalkoxy und/oder Halogen substituiertes Benzoyloxy. Niederalkanoyloxy ist z. B. Acetoxy, Propionyloxy, Butyryloxy, Isobutyryloxy, Valeroyloxy, Caproyloxy oder Pivaloyloxy.

Aliphtische, cycloaliphatische, aromatische und araliphatische Reste $R_1$ bzw. $R_2$ sind in erster Linie gegebenenfalls substituierte aliphatische, cycloaliphatische, aromatische oder araliphatische Kohlenwasserstoffreste, wie entsprechendes Niederalkyl, Niederalkenyl, Cycloalkyl, Phenyl, Naphthyl oder Phenylniederalkyl. Substituenten sind z. B. Hydroxy, Niederalkoxy, Niederalkyl- oder Phenylthio, Niederalkan- oder Benzolsulfinyl, oder Niederalkan- oder Benzolsulfonyl, insbesondere von Niederalkyl $R_1$ sowie Niederalkyl $R_2$, ferner Niederalkyl, Niederalkoxy und/oder Halogen, insbesondere von Phenyl oder Phenylniederalkyl $R_1$. Heterocyclyl in einem heterocyclischen oder

heterocyclisch-aliphatischen Rest $R_1$ ist in erster Linie monocyclisches Heterocyclyl aromatischen Charakters mit einem Heteroatom, wie Sauerstoff, Schwefel oder Stickstoff, als Ringglied, wie Furyl, Thienyl oder Pyridyl. In einem heterocyclisch-aliphatischen Rest $R_1$ ist der aliphatische Teil z. B. ein entsprechender aliphatischer Kohlenwasserstoffrest, insbesondere Niederalkyl.

Niederalkyliden X ist beispielsweise Methylen.

Außer durch den Rest $R_1-X-$ kann 1,2-Phenylen zusätzlich, u. a. durch Niederalkyl, Niederalkoxy, Hydroxy und/oder Halogen, einfach oder mehrfach substituiert sein.

Niederalkoxy bedeutet z. B. Methoxy, Äthoxy, n-Propyloxy, Isopropyloxy, n-Butyloxy, Isobutyloxy, tert.-Butyloxy, n-Pentyloxy oder n-Hexyloxy.

Phenylniederalkoxy ist z. B. Benzyloxy oder 1- oder 2-Phenyläthoxy.

Hydroxy-, Niederalkoxy- bzw. Diniederalkylaminoniederalkoxy ist insbesondere 2- und/oder 3-Hydroxy-niederalkoxy, z. B. 2-Hydroxyäthoxy, 3-Hydroxypropyloxy oder 2,3-Dihydroxy-propyloxy, sowie 2- oder 3-Niederalkoxy-niederalkoxy, z. B. 2-Methoxyäthoxy, 2-Äthoxyäthoxy oder 3-Methoxypropyloxy, bzw. Diniederalkylaminoniederalkoxy, z. B. Dimethylamino- oder Diäthylaminoäthoxy.

Niederalkyl ist z. B. Methyl, Äthyl, n-Propyl, Isopropyl, n-Butyl, Isobutyl, tert.-Butyl, n-Pentyl, n-Hexyl oder n-Heptyl.

Halogen ist insbesondere Halogen mit Atomnummer bis und mit 35, d. h. Fluor, Chlor oder Brom.

Niederalkylen ist z. B. 1,4-Butylen, 1,5-Pentylen oder 1,6-Hexylen.

Niederalkenyl ist z. B. Vinyl, 1-Methyl-vinyl, 1-Äthyl-vinyl, Allyl, 2- oder 3-Methyl-allyl oder 3,3-Dimethyl-allyl.

Cycloalkyl enthält vorzugsweise 3 bis 8 Ringatome und ist z. B. Cyclopropyl, Cyclopentyl, Cyclohexyl, Cycloheptyl oder Cyclooctyl.

Niederalkylthio ist z. B. Methylthio oder Äthylthio, während Niederalkansulfinyl und Niederalkansulfonyl z. B. Methansulfinyl, Äthansulfinyl, Methansulfonyl oder Äthansulfonyl bedeuten.

Durch Niederalkylthio, Niederalkansulfinyl oder Niederalkansulfonyl substituiertes Niederalkyl ist z. B. Methylthio- oder Äthylthiomethyl, 1- oder 2-Methylthio- oder 1- oder 2-Äthylthioäthyl, oder 2- oder 3-Methylthio- oder 2- oder 3-Äthylthiopropyl, Methansulfinyl- oder Äthansulfinylmethyl, 1- oder 2-Methansulfonyl- oder 1- oder 2-Äthansulfonyl-äthyl, oder 2- oder 3-Methansulfonyl oder 2- oder 3-Äthansulfonylpropyl. Durch Phenylthio, Benzolsulfinyl oder Benzolsulfonyl substituiertes Niederalkyl ist z. B. Phenylthio-, Benzolsulfinyl- oder Benzolsulfonylmethyl, oder 1- oder 2-Phenylthio-, 1- oder 2-Benzolsulfinyl-, oder 1- oder 2-Benzolsulfonyläthyl.

Phenylniederalkyl ist z. B. Benzyl, 1- oder 2-Phenyläthyl oder 1-, 2- oder 3-Phenylpropyl.

Furyl ist z. B. 2-Furyl, und Thienyl z. B. 2-Thienyl, während Pyridyl 2-, 3- oder 4-Pyridyl sein kann.

Furylniederalkyl, Thienylniederalkyl und Pyridylniederalkyl sind insbesondere entsprechend substituierte Methylreste, wie Furfuryl, 2-Thenyl oder Picolyl, z. B. 2- oder 4-Pyridylmethyl.

Salze sind z. B. solche von Verbindungen der Formel I, worin R für Carboxy steht, mit Basen. Solche Salze sind insbesondere pharmazeutisch verwendbare, nicht-toxische Salze, wie Alkalimetall- oder Erdalkalimetall-, z. B. Natrium-, Kalium-, Magnesium- oder Calciumsalze, ferner Ammoniumsalze mit Ammoniak oder Aminen, wie Niederalkylaminen, z. B. Trimethylamin oder Triäthylamin, oder mineralsaure Salze von Verbindungen der Formel I mit basischer Seitenkette, z. B. entsprechende Hydrohalogenide, wie -chloride.

In der britischen Patentschrift Nr. 766 749 sind eine Reihe strukturähnlicher Verbindungen, namentlich 5-Methyl- und 5,6-Dimethylbenzimidazol-2-derivate, die als Substituent in 2-Stellung Carboxy, Carbamyl oder Hydroxymethyl aufweisen, als Ausgangsstoffe für die Herstellung von Benzimidazolcobalaminen beschrieben. Ferner wurden in der US-Patentschrift Nr. 3 325 271 u. a. im Benzoteil alkylierte 2-Alkoxyalkyl-benzimidazole als Pflanzenschutzmittel vorgeschlagen und in der bekanntgemachten niederländischen Patentanmeldung Nr. 7 004 376 im Benzoteil alkylierte Benzimidazol-2-carboxyamide als Zwischenprodukte beschrieben. Überraschenderweise wurde nun festgestellt, daß die Verbindungen der Formel I wertvolle pharmakologische Eigenschaften aufweisen. Insbesondere zeigen sie antiallergische Wirkungen, die z. B. an der Ratte in Dosen von etwa 10 bis etwa 100 mg/kg bei oraler Verabreichung im passiven kutanen Anaphylaxie-Test (PCA-Reaktion), der analog der von Goose und Blair, Immunology, Bd. 16, S. 749 (1969) beschriebenen Methode durchgeführt wird, nachgewiesen werden können, wobei die passive kutane Anaphylaxie nach dem von Ovary, Prog. Allergy, Bd. 5, S. 459 (1958), beschriebenen Verfahren erzeugt wird. Die antiallergische, insbesondere die degranulationshemmende Wirkung kann in einem in vitro-Versuch auch anhand der Histaminfreisetzung aus Peritonealzellen der Ratte bei immunologisch induzierter Freisetzung (wobei z. B. mit Nippostrongylus brasiliensis infestierte Ratten verwendet werden) und bei chemisch induzierter Freisetzung (wobei diese z. B. mit einem Polymeren von N-4-Methoxy-phenyläthyl-N-methyl-amin bewirkt wird) festgestellt werden. Die Verbindungen der vorliegenden Erfindung sind deshalb als Hemmer von allergischen Reaktionen, z. B. in der Behandlung und Prophylaxe von allergischen Erkrankungen, wie Asthma, sowohl extrinsic als auch intrinsic Asthma, oder anderen allergischen Erkrankungen, wie allergischer Rhinitis, z. B. Heufieber, Konjunktivitis, oder allergischer Dermatis, z. B. Urticaria oder Ekzeme verwendbar.

Die Erfindung betrifft insbesondere solche der vorstehend definierten Verbindungen der Formel I, worin R für freies Carboxy oder Hydroxymethyl, als verätherte Hydroxygruppe Niederalkoxy,

Hydroxyniederalkoxy, Niederalkoxyniederalkoxy oder Diniederalkylaminoniederalkoxy aufweisendes verestertes Carboxy oder veräthertes Hydroxymethyl, als Aminogruppe Amino, Hydroxyamino, Niederalkylamino, Diniederalkylamino oder Niederalkylenamino aufweisendes amidiertes Carboxy oder als veresterte Hydroxygruppe Niederalkanoyloxy oder gegebenenfalls durch Niederalkyl, Niederalkoxy und/oder Halogen substituiertes Benzoyloxy aufweisendes verestertes Hydroxymethyl steht, $R_1$ gegebenenfalls durch Niederalkoxy, Niederalkylthio, Niederalkansulfinyl, Niederalkansulfonyl, Phenylthio, Benzolsulfinyl oder Benzolsulfonyl substituiertes Niederalkyl, Niederalkenyl, Cycloalkyl, gegebenenfalls im Phenylteil durch Niederalkyl, Niederalkoxy oder Halogen substituiertes Phenyl oder Phenylniederalkyl, Furyl, Thienyl oder Pyridyl, oder Furylniederalkyl, Thienylniederalkyl oder Pyridylniederalkyl bedeutet, X Methylen bedeutet, $R_2$ Wasserstoff oder Niederalkyl darstellt, und Ph für den Rest $R_1-X-$ enthaltende und gegebenenfalls durch Niederalkyl, Niederalkoxy, Hydroxy und/oder Halogen substituiertes 1,2-Phenylen steht, z. B. solche, in denen $R_1$ mindestens 2 Kohlenstoffatome aufweist, wenn Ph ansonsten unsubstituiert ist, $R_2$ Äthyl bedeutet und R Acetoxymethyl darstellt, und pharmazeutisch verwendbare Salze der genannten Verbindungen, worin R für Carboxy steht, Verfahren zu ihrer Herstellung, diese enthaltende pharmazeutische Präparate und ihre Anwendung in einem Verfahren zur prophylaktischen und/oder therapeutischen Behandlung des menschlichen oder tierischen Körpers.

Die Erfindung betrifft insbesondere solche der vorstehend definierten Verbindungen der Formel I, worin R für freies Carboxy, als verätherte Hydroxygruppe Niederalkoxy oder Hydroxyniederalkoxy mit bis und mit 4 Kohlenstoffatomen, z. B. Methoxy, Äthoxy, 2-Hydroxyäthoxy oder 2,3-Dihydroxypropyloxy, aufweisendes verestertes Carboxy, oder als Aminogruppe Amino oder Hydroxyamino, Niederalkylamino oder Diniederalkylamino, worin Niederalkyl bis und mit 4 Kohlenstoffatome enthält, z. B. Methylamino, Äthylamino, Dimethylamino oder Diäthylamino, aufweisendes amidiertes Carboxy steht oder Hydroxymethyl, als verätherte Hydroxygruppe Niederalkoxy mit bis zu 4 Kohlenstoffatomen, z. B. Methoxy oder Äthoxy, oder Diniederalkylaminoniederalkoxy mit jeweils bis zu 4 Kohlenstoffatomen im Alkyl- bzw. Alkoxyteil, wie Dimethylaminoäthoxy, aufweisendes veräthertes Hydroxymethyl oder als veresterte Hydroxygruppe Niederalkanoyloxy mit bis zu 7 Kohlenstoffatomen, z. B. Acetoxy, Propionyloxy oder Pivaloyloxy, oder gegebenenfalls durch Niederalkyl, z. B. Methyl, Niederalkoxy, z. B. Methoxy und/oder Halogen, z. B. Chlor substituiertes Benzoyloxy aufweisendes verestertes Hydroxymethyl bedeutet, $R_1$ Niederalkyl mit bis und mit 7 Kohlenstoffatomen, z. B. Methyl, Äthyl, n-Propyl, Isopropyl, n-Butyl, Isobutyl, tert.-Butyl, n-Pentyl, Neopentyl, n-Hexyl oder n-Heptyl, Niederalkoxy-, Niederalkylthio-, Niederalkansulfinyl- oder Niederalkansulfonyl-niederalkyl, worin die einzelnen Niederalkylreste bis und mit 4 Kohlenstoffatome enthalten, z. B. Methoxy-, Äthoxy-, Methylthio-, Äthylthio-, Methansulfinyl-, Äthansulfinyl-, Methansulfonyl- oder Äthansulfonylmethyl, 1- oder 2-Methoxy-, 1- oder 2-Äthoxy-, 1- oder 2-Methylthio-, 1- oder 2-Äthylthio-, 1- oder 2-Methansulfinyl-, 1- oder 2-Äthansulfinyl-, 1- oder 2-Methansulfonyl- oder 1- oder 2-Äthansulfonyläthyl, oder 1-, 2- oder 3-Methoxy-, 1-, 2- oder 3-Äthoxy-, 1-, 2- oder 3-Methylthio-, 1-, 2- oder 3-Äthylthio-, 1-, 2- oder 3-Methansulfinyl-, 1-, 2- oder 3-Äthansulfinyl-, 1-, 2- oder 3-Methansulfonyl- oder 1-, 2- oder 3-Äthansulfonylpropyl, Phenylthio-, Benzolsulfinyl- oder Benzolsulfonyl-niederalkyl, worin der Niederalkylrest bis und mit 4 Kohlenstoffatome enthält, z. B. Phenylthio-, Benzolsulfinyl- oder Benzolsulfonylmethyl, 1- oder 2-Phenylthio-, 1- oder 2-Benzolsulfinyl- oder 1- oder 2-Benzolsulfonyläthyl, oder 1-, 2- oder 3-Phenylthio-, 1-, 2- oder 3-Benzolsulfinyl- oder 1-, 2- oder 3-Benzolsulfonylpropyl, Niederalkenyl mit bis und mit 5 Kohlenstoffatomen, z. B. 1-Methyl- oder 1-Äthyl-vinyl oder Allyl, Cycloalkyl mit bis und mit 7 Kohlenstoffatomen, z. B. Cyclopropyl oder Cyclohexyl, gegebenenfalls durch Niederalkyl mit bis und mit 4 Kohlenstoffatomen, z. B. Methyl, Niederalkoxy mit bis und mit 4 Kohlenstoffatomen, z. B. Methoxy, und/oder Halogen mit Atomnummer bis und mit 35, z. B. Chlor oder Brom, substituiertes Phenyl oder Phenylniederalkyl mit bis und mit 4 Kohlenstoffatomen im Niederalkylrest, z. B. Benzyl oder 1- oder 2-Phenyläthyl, Furyl, Thienyl oder Pyridyl, z. B. 2-Furyl, 2-Thienyl oder 2-, 3- oder 4-Pyridyl, oder Furyl-, Thienyl- oder Pyridyl-niederalkyl mit bis und mit 4 Kohlenstoffatomen im Niederalkylrest, z. B. Furfuryl, 2-Thenyl oder 2- oder 4-Picolyl, bedeutet, X Methylen bedeutet, $R_2$ Wasserstoff oder Niederalkyl mit bis und mit 4 Kohlenstoffatomen, z. B. Methyl, darstellt, und Ph das den Rest der Formel $R_1-X-$ enthaltende, gegebenenfalls durch Niederalkyl mit bis und mit 4 Kohlenstoffatomen, z. B. Methyl, Niederalkoxy mit bis zu und mit 4 Kohlenstoffatomen, z. B. Methoxy, Hydroxy und/oder Halogen mit Atomnummer bis und mit 35, z. B. Chlor oder Brom, substituiertes 1,2-Phenylen darstellt, wobei der Rest der Formel $R_1-X-$ irgendeine zur Substitution geeignete Stellung, vorzugsweise die 4- oder 5-Stellung des 1,2-Phenylenrestes einnimmt, z. B. solche, in denen $R_1$ mindestens 2 Kohlenstoffatome aufweist, wenn Ph ansonsten unsubstituiert ist, $R_2$ Äthyl bedeutet und R Acetoxymethyl darstellt, und pharmazeutisch verwendbare Salze der genannten Verbindungen, worin R für Carboxy steht, mit Basen, Verfahren zu ihrer Herstellung, diese enthaltende pharmazeutische Präparate und ihre Anwendung in einem Verfahren zur prophylaktischen und/oder therapeutischen Behandlung des menschlichen oder tierischen Körpers.

Die Erfindung betrifft in erster Linie Verbindungen der Formel

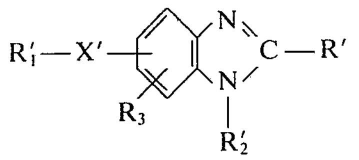

(Ia)

worin R′ einerseits in erster Linie für Carboxy oder ferner für als verätherte Hydroxygruppe Niederalkoxy mit bis und mit 4 Kohlenstoffatomen, z. B. Methoxy oder Äthoxy, aufweisendes verestertes Carboxy steht und andrerseits in erster Linie Hydroxymethyl oder ferner als verätherte Hydroxygruppe Niederalkoxy mit bis zu 4 Kohlenstoffatomen, z. B. Methoxy oder Äthoxy, aufweisendes veräthertes Hydroxymethyl darstellt, und worin $R'_1$ Niederalkyl mit bis und mit 7 Kohlenstoffatomen, z. B. Methyl, Äthyl, n-Propyl, n-Butyl, tert.-Butyl, Cycloalkyl mit bis und mit 6 Ringkohlenstoffatomen, z. B. Cyclopropyl oder Cyclohexyl oder Phenyl darstellt, X′ Methylen bedeutet, $R'_2$ für Wasserstoff oder Niederalkyl mit bis zu 4 Kohlenstoffatomen, z. B. Methyl, steht, und $R_3$ Wasserstoff, Niederalkyl mit bis und mit 4 Kohlenstoffatomen, z. B. Methyl, Niederalkoxy mit bis und mit 4 Kohlenstoffatomen, z. B. Methoxy, oder Halogen mit Atomnummer bis und mit 35, z. B. Chlor, bedeutet, wobei der Rest der Formel $R'_1-X'-$ und die Gruppe $R_3$, falls diese von Wasserstoff verschieden ist, vorzugsweise die 5- und die 6-Stellung des Benzimidazolrings einnehmen, und pharmazeutisch verwendbare Salze von Verbindungen der Formel Ia, in denen R′ für Carboxy steht, mit Basen zur Anwendung in einem Verfahren zur prophylaktischen und/oder therapeutischen Behandlung des menschlichen oder tierischen Körpers, diese enthaltende pharmazeutische Präparate sowie Verbindungen der Formel Ia, mit der Maßgabe, daß R′ von als verätherte Hydroxygruppe Niederalkoxy mit bis und mit 4 Kohlenstoffatomen aufweisende Niederalkoxymethyl verschieden ist, wenn $R'_1$ Niederalkyl mit bis und mit 6 Kohlenstoffatomen bedeutet, und pharmazeutisch verwendbare Salze von Verbindungen der Formel Ia, worin R′ Carboxy bedeutet, mit Basen selbst und Verfahren zu ihrer Herstellung.

Die Erfindung betrifft in erster Linie solche der vorstehend definierten Verbindungen der Formel Ia, worin R′ für Carboxy oder für als verätherte Hydroxygruppe Niederalkoxy mit bis und mit 4 Kohlenstoffatomen, z. B. Methoxy oder Äthoxy, aufweisendes verestertes Carboxy steht, und worin $R'_1-X'$ Niederalkyl mit 2 bis 7 Kohlenstoffatomen, z. B. Äthyl, n-Propyl, Isopropyl, n-Butyl, n-Pentyl oder n-Hexyl, Cycloalkylmethyl mit bis und mit 6 Ringkohlenstoffatomen, z. B. Cyclopropyl- oder Cyclohexylmethyl, oder Benzyl bedeutet, $R'_2$ für Wasserstoff oder insbesondere für Niederalkyl mit bis und mit 4 Kohlenstoffatomen, z. B. Methyl, steht, $R_3$ Wasserstoff oder Niederalkyl mit bis zu 4 Kohlenstoffatomen, z. B. Methyl, Niederalkoxy mit bis und mit 4 Kohlenstoffatomen, z. B. Methoxy, oder Halogen mit Atomnummer bis und mit 35, z. B. Chlor, bedeutet, wobei die Reste $R'_1-X'-$ und $R_3$ vorzugsweise die 5- bzw. 6-Stellung des Benzimidazolrings einnehmen, und pharmazeutisch verwendbare Salze von Verbindungen der Formel Ia in denen R′ für Carboxy steht, mit Basen zur Anwendung in einem Verfahren zur prophylaktischen und/oder therapeutischen Behandlung des menschlichen oder tierischen Körpers, diese enthaltende pharmazeutische Präparate, sowie Verbindungen der Formel Ia und pharmazeutisch verwendbare Salze dieser Verbindung mit salzbildenden Eigenschaften, und Verfahren zu ihrer Herstellung.

Die Erfindung betrifft in allererster Linie solche der vorstehend definierten Verbindungen der Formel Ia, worin R′ Carboxy, Hydroxymethyl oder Niederalkoxycarbonyl oder Niederalkoxymethyl mit insgesamt bis zu 5 Kohlenstoffatomen, z. B. Methoxy- oder Äthoxycarbonyl oder -methyl, bedeutet, $R'_1-X'$ Niederalkyl mit bis zu 8, beispielsweise mit bis zu 5, Kohlenstoffatomen, z. B. Methyl, Äthyl, n-Propyl, Isopropyl, n-Butyl, Isobutyl oder n-Pentyl, bedeutet und $R'_2$ und $R_3$ unabhängig voneinander Wasserstoff oder Niederalkyl mit bis zu 4 Kohlenstoffatomen, wie Methyl, darstellen, und pharmazeutisch verwendbare Salze von Verbindungen der Formel Ia in denen R′ für Carboxy steht, mit Basen zur Anwendung in einem Verfahren zur prophylaktischen und/oder therapeutischen Behandlung des menschlichen oder tierischen Körpers, diese enthaltende pharmazeutische Präparate, sowie Verbindungen der Formel Ia und pharmazeutisch verwendbare Salze dieser Verbindungen mit salzbildenden Eigenschaften, worin R′ Carboxy bedeutet, mit Basen selbst, mit der Maßgabe, daß R von Niederalkoxymethyl mit bis und mit 5 Kohlenstoffatomen verschieden ist, wenn $R'_1-X'-$ Niederalkyl mit bis und mit 7 Kohlenstoffatomen bedeutet, und mit der weiteren Maßgabe, daß eine Methylgruppe $R'_1-X'-$ nicht die 5(6)-Stellung des Benzimidazolringes einnimmt, wenn $R_3$ Wasserstoff oder in 6(5)-Stellung gebundenes Methyl ist, $R'_2$ Wasserstoff und R′ Carboxy oder Hydroxymethyl darstellt, und Verfahren zu ihrer Herstellung.

Die Erfindung betrifft in allererster Linie z. B. Verbindungen der Formel Ia, worin R′ entweder für Carboxy oder für Niederalkoxycarbonyl mit insgesamt bis zu 5 Kohlenstoffatomen, wie Methoxy- oder Äthoxycarbonyl, steht, und worin $R'_1-X'-$ Niederalkyl mit bis und mit 7 Kohlenstoffatomen, z. B. Methyl, Äthyl, n-Propyl, Isopropyl, n-Butyl, n-Pentyl oder n-Hexyl, darstellt und $R'_2$ und $R_3$ Niederalkyl mit bis und mit 4 Kohlenstoffatomen, z. B. Methyl, darstellen, wobei der Rest $R'_1-X'-$ die 5- und der Niederalkylrest $R_3$ die 6-Stellung des Benzimidazolrings einnimmt, und Salze von solchen Verbindungen, in denen R′ Carboxy ist, mit Basen sowie Verfahren zu ihrer Herstellung, die genannten

Verbindungen und pharmazeutisch verwendbare Salze solcher Verbindungen, in denen R′ Carboxy bedeutet, mit Basen zur Anwendung in einem Verfahren zur prophylaktischen und/oder therapeutischen Behandlung des menschlichen oder tierischen Körpers und diese enthaltende pharmazeutische Präparate.

Die Erfindung betrifft in allererster Linie z. B. ferner Verbindungen der Formel Ia, worin R′ für Carboxy steht, $R'_1$ Niederalkyl mit bis zu 7, z. B. mit bis zu 4, Kohlenstoffatomen, wie Methyl, Äthyl, n-Propyl, Isopropyl oder n-Butyl, darstellt, X′ Methylen bedeutet, $R'_2$ für Wasserstoff steht und $R_3$ Wasserstoff oder Niederalkyl mit bis zu 4 Kohlenstoffatomen, wie Methyl, darstellt, wobei der Rest $R'_1-X'-$ die 5- und ein Niederalkylrest $R_3$ die 6-Stellung des Benzimidazolringes einnimmt, sowie Verfahren zu ihrer Herstellung, die genannten Verbindungen und pharmazeutisch verwendbare Salze solcher Verbindungen, in denen R′ Carboxy bedeutet, mit Basen zur Anwendung in einem Verfahren zur prophylaktischen und/oder therapeutischen Behandlung des menschlichen oder tierischen Körpers und diese enthaltende pharmazeutische Präparate.

Die Erfindung betrifft namentlich die in den Beispielen genannten Verbindungen der Formel 1.

Die neuen Verbindungen können in an sich bekannter Weise hergestellt werden. So kann man sie z. B. erhalten, indem man eine Verbindung der Formel II

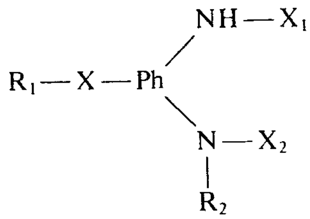

(II)

worin einer der Reste $X_1$ und $X_2$ eine Gruppe der Formel $-C(=O)-R$ und der andere Wasserstoff bedeutet, oder ein Salz davon cyclisiert, und, wenn erwünscht, eine so erhältliche Verbindung in eine andere Verbindung der Formel I umwandelt und/oder eine erhaltene freie salzbildende Verbindung in ein Salz oder ein erhaltenes Salz in die freie Verbindung oder in ein anderes Salz überführt.

Als Salze von Ausgangsstoffen der Formel II kommen beispielsweise Säureadditionssalze, wie Hydrohalogenide, z. B. die Hydrochloride, von Verbindungen, in denen R gegebenenfalls veräthertes oder verestertes Hydroxymethyl bedeutet, bzw. Alkalimetall- oder Ammoniumsalze, z. B. die Natriumsalze, von Verbindungen, in denen R Carboxy bedeutet, in Betracht.

Die Cyclisierung erfolgt in üblicher Weise, bei normaler oder insbesondere zur Herstellung von Verbindungen, in denen R gegebenenfalls veräthertes Hydroxymethyl bedeutet, erhöhter Temperatur, z. B. bei etwa 50°C bis etwa 160°C, vor allem bei etwa 110°C bis 140°C, erforderlichenfalls in Gegenwart eines sauren Kondensationsmittels, wie einer Halogenwasserstoffsäure, z. B. von Salzsäure, und/oder eines wasserbindenden Mittels, z. B. von Dicyclohexylcarbodiimid, und vorteilhaft unter Inertgas, z. B. unter Stickstoff.

Die vorstehende Verfahrensvariante ist insbesondere geeignet zur Herstellung von Verbindungen der Formel I, in denen R gegebenenfalls veräthertes Hydroxymethyl bedeutet, die anschließend bequem in üblicher Weise in andere Verbindungen der Formel I überführt werden können.

Die Ausgangsstoffe der Formel II werden zweckmäßigerweise in situ hergestellt, beispielsweise indem man ein entsprechendes 1,2-Phenylendiamin, das durch den Rest $R_1-X-$ substituiert ist und gegebenenfalls noch weitere Substituenten enthalten kann, d. h. eine Verbindung der Formel

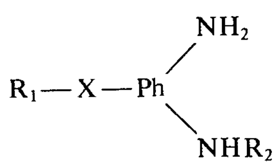

(IIa)

oder ein Säureadditionssalz davon, z. B. dessen Hydrochlorid, mit einer Säure der Formel $R-COOH$ (IIb) oder einem geeigneten reaktionsfähigen Derivat, vorzugsweise Ester, wie Niederalkylester, Amid, Anhydrid, wie Säurehalogenid, Iminoäther, wie Iminoniederalkyläther oder Iminoester, wie Iminochlorid, davon, z. B. mit der vorzugsweise verätherten Glykolsäure oder mit Chlor- bzw. Bromoxalsäureäthylester, umsetzt, erforderlichenfalls in Gegenwart eines Lösungs- oder Verdünnungsmittels, wie eines Niederalkanols, z. B. von Methanol oder Äthanol, und/oder unter Erwärmen auf etwa 50°C bis etwa 160°C, z. B. auf etwa 110°C bis etwa 140°C. Die dafür als Ausgangsstoffe zu verwendenden 1,2-Phenylendiamine können beispielsweise durch übliche Reduktion, z. B. Umsetzung mit einem chemischen Reduktionsmittel, wie Natriumdithionit, oder mit geeignet aktiviertem Wasserstoff, wie durch einen Edelmetallkatalysator in basischem Milieu, z. B. durch Raney-Nickel in Methanol oder Äthanol, katalytisch aktivierten Wasserstoff, der entsprechenden 1,2-Nitranilinverbindung erhalten werden. In einer Abwandlung dieser Methode kann man auch dieses 1,2-Nitranilin-zwischenprodukt mit der obenerwähnten Säure, z. B. mit Glykol- oder Oxalsäure, oder mit einem

geeigneten Derivat davon, z. B. mit einem Äthoxyessigsäure- oder Chloroxalsäureniederalkylester, umsetzen und anschließend die Nitrogruppe, z. B. mit Wasser in Gegenwart von Raney-Nickel, reduzieren.

Die für die Herstellung der Ausgangsstoffe der Formel II zu verwendenden 1,2-Nitranilinverbindungen können, sofern sie nicht bekannt sind, z. B. ausgehend von den entsprechenden Chlorbenzolen der Formel H – PhH – Cl hergestellt werden, indem man diese in üblicher Weise, z. B. durch Umsetzung mit einer Verbindung der Formel $R_1 - X - Hal$ oder $R_1 - X - OH$ bzw. mit einem entsprechenden Alken oder Cycloalken in Gegenwart von Aluminiumchlorid substituiert, die so erhältliche Verbindung der Formel $R_1 - X - PhH - Cl$ mit Salpetersäure/Schwefelsäure nitriert und die so erhältliche Chlornitroverbindung der Formel $R_1 - X - Ph(Cl) - NO_2$ mit Ammoniak oder einem Amin der Formel $R_2NH_2$ umsetzt.

Eine bevorzugte Ausführungsform des vorstehend beschriebenen Verfahrens besteht darin, daß man eine Verbindung der Formel

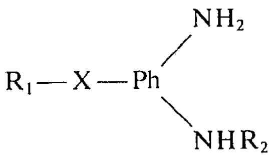

(IIa)

mit einer Säure der Formel R – COOH (IIb) oder einem geeigneten funktionellen Derivat davon umsetzt, und, wenn erwünscht, eine so erhältliche Verbindung in eine andere Verbindung der Formel I umwandelt und/oder eine erhaltene freie salzbildende Verbindung in ein Salz oder ein erhaltenes Salz in die freie Verbindung oder in ein anderes Salz überführt.

Geeignete funktionelle Derivate von Säuren der Formel IIb sind dabei z. B. deren Ester, wie Niederalkylester, Amide oder Anhydride, wie Säurehalogenide. Als in der vorstehenden Verfahrensvariante einzusetzende Säuren der Formel IIb und deren funktionelle Derivate kommen beispielsweise die gegebenenfalls verätherte Glyoxylsäure und Chlor- bzw. Bromoxalsäureäthylester in Betracht.

Die Umsetzung erfolgt insbesondere in Gegenwart eines Lösungs- oder Verdünnungsmittels, wie eines Niederalkanols, z. B. von Methanol oder Äthanol, erforderlichenfalls unter Erwärmen auf etwa 50° C bis 160° C, z. B. auf etwa 110° C bis etwa 140° C.

Die neuen Verbindungen können ferner hergestellt werden, indem man in einer Verbindung der Formel

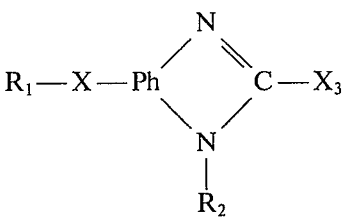

(III)

worin $X_3$ einen in die Gruppe R überführbaren Rest darstellt, $X_3$ in die Gruppe R überführt, und, wenn erwünscht, eine so erhältliche Verbindung der Formel I in eine andere Verbindung der Formel I umwandelt und/oder, wenn erwünscht, ein erhaltenes Salz in die freie Verbindung oder in ein anderes Salz und/oder eine freie salzbildende Verbindung in ein Salz überführt.

Eine Gruppe $X_3$ ist in erster Linie ein oxydativ in die Carboxygruppe oder reduktiv in Hydroxymethyl R überführbarer Rest, und insbesondere die Formylgruppe, wobei diese auch in situ im Verlauf der Oxidationsreaktion, z. B. aus der Methyl- oder Aminomethylgruppe oder einer mit einer anorganischen Säure, wie einer Halogenwasserstoffsäure, z. B. mit Chlorwasserstoffsäure, veresterten oder mit einem cyclischen 2-Hydroxyäther, z. B. mit 2-Hydroxytetrahydropyran, oder einem cyclischen 2- oder 4-Hydroxythioäther, z. B. mit 2-Hydroxytetrahydrothiopyran, 2-Hydroxytetrahydrothiophen oder 4-Hydroxy-4-methoxy-tetrahydrothiopyran, verätherten Hydroxymethylgruppe, gebildet oder aus einem ihrer Derivate, wie einem Niederalkylen- oder Diniederalkylacetal oder Imino, z. B. Benzylimin bzw. Imminiumsalz, wie N,N-Diniederalkyl-, z. B. N,N-Dimethylimminiumsalz, z. B. -chlorid oder -methosulfat, in Freiheit gesetzt werden kann. Ferner kann auch gegebenenfalls substituiertes, z. B. in 5-Stellung Diniederalkoxymethyl, enthaltendes, 2-Furyl oxidativ in die Carboxylgruppe übergeführt werden.

Die Oxidation kann in an sich bekannter Weise, z. B. durch Behandeln mit einer oxidierenden Schwermetallverbindung, bei Ausgangsstoffen der Formel II, worin $X_3$ für die Formylgruppe oder einen oxidativ in diese überführbaren Rest, wie eine der genannten veresterten oder verätherten Hydroxymethylgruppen, oder für gegebenenfalls substituiertes 2-Furyl steht, vorzugsweise mit einer Chrom-VI- oder Mangan-VII-enthaltenden, oxidierenden Verbindung, z. B. Chromtrioxid oder insbesondere Kaliumpermanganat, und bei Ausgangsstoffen der Formel II, worin $X_3$ für eine der genannten verätherte Hydroxymethylgruppen steht, ferner mit einer Mangan-IV-enthaltenden,

oxidierenden Verbindung, wie Mangandioxid, durchgeführt werden. Dabei arbeitet man vorteilhafterweise in Gegenwart eines geeigneten Lösungs- oder Verdünnungsmittels, z. B. von Aceton oder Pyridin, oder eines, vorzugsweise wäßrigen, Gemisches davon, wenn notwendig, unter Kühlen oder Erwärmen, z. B. in einem Temperaturenbereich von etwa 0° C bis etwa 80° C.

Eine Formylgruppe kann z. B. auch durch Reduktion mit einem Leichtmetall- oder Dileichtmetallhydrid, wie einem Bor-, Natriumbor- oder Lithiumaluminiumhydrid, z. B. mit Natriumborhydrid oder mit Natriumcyanoborhydrid in einem Niederalkanol, Lithiumaluminiumhydrid in Äther, oder Diisoamylboran in Tetrahydrofuran, erforderlichenfalls unter Kühlen oder gelindes Erwärmen, z. B. bei etwa 0° C bis etwa 100° C, und/oder unter Inertgas, wie Stickstoff zu Hydroxymethyl reduziert werden.

Weitere, in die Gruppen der Formel R überführbare Reste $X_3$ sind von der gegebenenfalls veresterten oder amidierten Carboxylgruppe der Formel R verschiedene und in diese überführbare funktionell abgewandelte Carboxylgruppe, wie Cyano, Halogen-, z. B. Chlorcarbonyl, reaktive veresterte Carboxylgruppen, wie Mono-, Di- oder Trihalogen-, z. B. Chlor-, Di-chlor- oder Trichloräthoxycarbonyl, Phenoxy- oder 4-Nitrophenoxy- bzw. 2,4-Dinitrophenoxycarbonyl, oder reaktive Carbamylgruppen, wie Imidazolyl-2-carbonyl, offenkettige oder cyclische Iminoäthergruppen, z. B. Iminoniederalkyläthergruppen oder 4,4- oder 5,5-Dimethyl-4,5-dihydrooxazolyl-(2), oder 4,4,6-Trimethyl-5,6-dihydro-oxazinyl-(2), oder Triniederalkoxy- oder Trihalogenmethylgruppen, z. B. Trichlormethyl. Diese Gruppen können solvolytisch, z. B. hydrolytisch, üblicherweise in Gegenwart eines sauren oder vorzugsweise alkalischen Hydrolysemittels, wie einer organischen Sulfonsäure, z. B. p-Toluolsulfonsäure oder Mesitylensulfonsäure, oder einer Mineralsäure, z. B. Schwefelsäure, oder eines Alkalimetallhydroxids, z. B. Natriumhydroxid, in die Carboxygruppe, eine Triniederalkoxymethyl- oder Iminoäthergruppe auch in verestertes Carboxy oder eine Cyanogruppe $X_3$ auch in die Carbamylgruppe übergeführt werden. Bei der Hydrolyse von Cyano und Trihalogenmethyl arbeitet man vorzugsweise basisch, bei der Hydrolyse von Iminoäthergruppen vorzugsweise sauer. Durch Behandeln eines Ausgangsmaterials der Formel II, worin $X_3$ für Halogencarbonyl oder eine der genannten reaktiven veresterten Carboxygruppen, mit einem Alkohol, z. B. einem Niederalkanol, Ammoniak oder einem entsprechenden Amin, erforderlichenfalls in Gegenwart eines basischen Mittels, z. B. von Pyridin, gelangt man zu Verbindungen der Formel I, worin R für eine veresterte oder amidierte Carboxygruppe, z. B. Niederalkoxycarbonyl, steht. Setzt man ein Ausgangsmaterial der Formel II, worin $X_3$ für Trihalogenmethyl, insbesondere Trichlormethyl steht, mit einem Alkohol, wie einem Niederalkanol, oder mit Ammoniak, Hydrolxylamin oder einem primären oder sekundären Amin und anschließend mit Wasser um, kann man direkt eine Verbindung der Formel I bilden, worin R für verestertes oder amidiertes Carboxy steht. Die genannten reaktiven veresterten Carboxylgruppen und Carbamylgruppen können ebenfalls durch Umsetzung mit Ammoniak oder einem entsprechenden Amin zu amidierten Carboxygruppen solvolysiert werden. Die obigen Reaktionen können nach an sich bekannten Methoden, üblicherweise in Gegenwart eines Lösungs- oder Verdünnungsmittels oder eines Gemisches davon, und, wenn notwendig, unter Kühlen oder Erwärmen, z. B. einem Temperaturenbereich von etwa 0° C bis etwa 120° C, durchgeführt werden.

Weitere solvolytisch in Gruppen der Formel R überführbare Reste $X_3$ sind beispielsweise von gegebenenfalls verestertem oder veräthertem Hydroxymethyl verschiedene und solvolytisch in diese überführbare veresterte oder verätherte Hydroxymethylgruppen. Derartige veresterte Hydroxymethylgruppen sind beispielsweise mit anorganischen Säuren, wie Halogenwasserstoffsäuren veresterte Hydroxymethylgruppen, z. B. Chlor- oder Brommethyl. Verätherte Hydroxymethylgruppen der vorstehend definierten Art sind beispielsweise mit einem cyclischen, vorzugsweise 5- oder 6-gliedrigen 2- oder 4-Hydroxyäther oder thioäther, z. B. mit 2-Hydroxytetrahydropyran, 2-Hydroxytetrahydrothiopyran, 2-Hydroxytetrahydrothiophen oder 4-Hydroxy-4-methoxy-tetrahydrothiopyran, oder mit einem Silanol, wie einem Triniederalkylsilanol, z. B. mit Trimethylsilanol, verätherte Hydroxymethylgruppen. Die genannten Gruppen können in üblicher Weise, beispielsweise in Gegenwart eines sauren oder vor allem basischen Hydrolysemittels, wie einer organischen Sulfonsäure, z. B. von p-Toluolsulfonsäure oder Mesitylensulfonsäure, oder einer Mineralsäure, z. B. von Schwefelsäure oder Chlorwasserstoffsäure, oder eines Alkalimetallhydroxides, z. B. von Natriumhydroxid, zu Hydroxymethyl hydrolysiert werden. Bei der Hydrolyse von veresterten Hydroxymethylgruppen arbeitet man vorzugsweise basisch, z. B. in Natronlauge. Die Hydrolyse von mit Hydroxyäthern oder Hydroxythioäthern verätherten Hydroxymethylgruppen wird vorzugsweise mild-sauer bewirkt, z. B. mittels p-Toluolsulfonsäure in Methanol oder Toluol. Thioätherverbindungen können in Gegenwart von Silbersalze, wie Silbernitrat, auch neutral hydrolysiert werden. Für die Hydrolyse von Silanyloxymethylgruppen sind keine Hilfsmittel erforderlich. In der angegebenen Art veresterte Hydroxymethylgruppen können ferner durch Umsetzung mit einem entsprechenden Alkohol, wie einem Niederalkanol, oder vorzugsweise einem entsprechenden Matallalkoholat, wie einem Alkalimetall-, z. B. dem Natriumniederalkanolat, zu verätherten Hydroxymethylgruppen R solvolysiert werden.

Die Ausgangsstoffe können in an sich bekannter Weise hergestellt werden.

Ausgangsstoffe der Formel II, in denen $X_3$ eine cyclische Iminoäthergruppe oder eine mit einem cyclischen 2- oder 4-Hydroxyäther oder 2- oder 4-Hydroxythioäther verätherte Hydroxymethylgruppe bedeutet, können beispielsweise hergestellt werden, indem man eine entsprechende Verbindung der

Formel

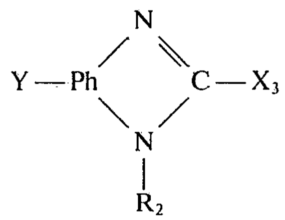

(IIIa)

worin Y eine Gruppe $-M-Hal$ oder $-M/2$, M ein Metallatom der Gruppe II des periodischen Systems der Elemente und Hal Chlor, Brom oder Jod bedeutet, mit einem Halogenid der Formel $R_1-X-Hal$ umsetzt und das Primärprodukt in üblicher Weise hydrolysiert. Die dafür als Ausgangsstoffe verwendeten Verbindungen der Formel IIIa werden vorteilhaft in situ hergestellt, indem man in einer Verbindung der Formel

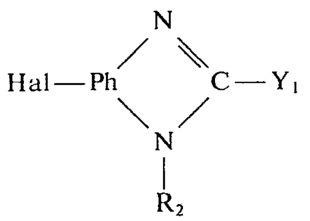

(IIIb)

worin Hal Chlor, Brom oder Jod und $Y_1$ Carboxy, Cyano oder Hydroxymethyl bedeutet, zunächst die Gruppe $Y_1$ in einen Rest $X_3$ überführt und anschließend Hal durch Umsetzung mit dem entsprechenden Metall, z. B. mit Magnesium, in die Gruppe Y, z. B. der Formel $-MgHal$, umwandelt. Verbindungen der Formel IIIa, in denen Y von Magnesium verschieden ist und z. B. eine Gruppe $-Cd/2$ bedeutet, können ferner durch Umsetzung der entsprechenden Halogenmagnesiumverbindungen mit einem Salz der Formel $MHal_2$, z. B. mit Cadmiumchlorid, hergestellt werden. Die Überführung von $Y_1$ in die genannten Gruppen erfolgt in üblicher Weise. Carboxy kann beispielsweise entweder zunächst, z. B. mittels Thionylchlorid in Methylenchlorid, in Halogencarbonyl überführt und anschließend durch Umsetzung mit dem betreffenden Aminoalkanol, z. B. mit Aminoisobutanol, oder durch Umsetzung mit dem betreffenden Aziridin, z. B. mit 2,2-Dimethylaziridin, und anschließende säurekatalysierte Ringerweiterung, in eine der genannten Iminoäthergruppen, z. B. in 4,4- bzw. 5,5-Dimethyl-4,5-dihydro-oxazolyl-(2)- überführt werden. Cyano kann durch Umsetzung mit dem betreffenden Aminoalkanol oder Alkandiol, z. B. mit 4-Amino-2-methyl-pentan-2-ol oder 2-Methyl-pentan-2,4-diol, unter Säurekatalyse ebenfalls in eine Iminoäthergruppe, z. B. in 4,4,6-Trimethyl-5,6-dihydro-oxazinyl-(2), überführt werden. Hydroxymethyl kann beispielsweise durch Umsetzung mit einem Chlorsilan, z. B. mit Trimethylchlorsilan in Pyridin, veräthert oder mit einem entsprechenden ungesättigten cyclischen Äther bzw. Thioäther, z. B. mit Dihydropyran, Dihydrothiopyran, 2,3-Dihydrothiophen oder 4-Methoxy-1,2-dihydro-$\gamma$-thipyran, unter Säurekatalyse, z. B. mit p-Toluolsulfonsäure in Toluol oder Aceton, in mit einem cyclischen 2- oder 4-Hydroxyäther oder -thioäther veräthertes Hydroxymethyl, z. B. in 2-Tetrahydropyranyloxymethyl, 2-Tetrahydrothiopyranyloxymethyl, 2-Tetrahydrothienyloxymethyl oder 4-(4-Methoxy)-tetrahydropyranyloxymethyl, überführt werden. Analog kann man auch Verbindungen der Formel III, in denen $X_3$ Formyl ist, herstellen, indem man in einer Verbindung der Formel IIIb, in der $Y_1$ acetalisiertes Formyl, wie Niederalkylendioxy- oder Diniederalkoxymethyl ist, Hal in eine Gruppe Y und anschließend in eine Gruppe $R_1-X-$ überführt und die acetalisierte Formylgruppe, z. B. säurekatalytisch, hydrolysiert.

Andere Ausgangsstoffe der Formel III können ausgehend von den entsprechenden 1,2-Phenylendiaminen, die durch den Rest der Formel $R_1-X-$ substituiert sind und gegebenenfalls weitere Substituenten enthalten können und die aus den entsprechenden Nitroanilinoverbindungen durch Reduktion der Nitrogruppe, z. B. mit Wasserstoff in Gegenwart von Raney-Nickel, zugänglich sind, in analoger Weise hergestellt werden, wie für ihre Behandlung mit Glykolsäure oder einem geeigneten reaktionsfähigen Derivat davon beschrieben, z. B. durch Umsetzung mit einer Säure der Formel $X_3-COOH$, wie einer Mono- oder Trihalogenessigsäure, Diniederalkoxyessigsäure oder 5-Dinederalkoxymethylfuran-2-carbonsäure, oder einem reaktiven Derivat, wie einem Niederalkylester davon.

Ferner kann man Ausgangsstoffe der Formel III, worin $X_3$ für Formyl oder Cyan steht, auch erhalten, indem man ein in 1- und 2-Stellung unsubstituiertes, im carbocyclischen Ring die Gruppe $R_1-X-$ enthaltendes und gegebenenfalls weiter substituiertes Benzimidazol mit 2-Chlor-1,1,2-trifluor-äthen umsetzt, und das so erhältliche, in 2-Stellung unsubstituierte 1-(2-Chlor-1,1,2-trifluoräthyl)-benzimidazol, das im carbocyclischen Teil die Gruppe $R_1$ und gegebenenfalls weitere Substituenten enthält, mit einem Alkohol, wie einem Niederalkanol, z. B. Äthanol, in Gegenwart einer Base, wie eines Alkalimetallhydroxids, z. B. Natriumhydroxid, oder mit einem Hydroxylamin-säureadditionssalz, z. B. dem Hydrochlorid, in Gegenwart einer Base, z. B. Pyridin, umsetzt. Man erhält so eine Verbindung der

Formel III, worin $X_3$ für eine acetalisierte Formylgruppe, wie Diniederalkoxymethyl, z. B. Diäthoxymethyl, bzw. die Hydroximinomethylgruppe steht, die man in an sich bekannter Weise, z. B. durch Hydrolyse in die Formylgruppe $X_3$ bzw. durch Dehydratisierung, z. B. mit Phosphorpentoxid oder 4-Methylphenylsulfonylchlorid, in die Cyanogruppe $X_3$ umwandeln kann.

Ein Ausgangsmaterial der Formel III, worin $X_3$ für Cyano steht, kann z. B. auch durch Behandeln einer Verbindung der Formel III, worin $X_3$ Trihalogenmethyl, z. B. Trichlormethyl, bedeutet, mit wäßrigem Ammoniak erhalten werden.

Die neuen Verbindungen können ferner hergestellt werden, indem man eine Verbindung der Formel

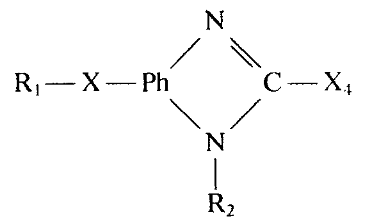

(IV)

mit einem reaktionsfähigen Derivat der Kohlensäure, z. B. mit Kohlendioxid oder einer Verbindung der Formel $R-X_5$ (V) umsetzt, worin $X_4$ ein Metallradikal und $X_5$ ein Halogenatom bedeutet, und wenn erwünscht, eine so erhältliche Verbindung der Formel I in eine andere Verbindung der Formel I umwandelt, und/oder, wenn erwünscht, ein erhaltenes Salz in die freie Verbindung oder in ein anderes Salz und/oder eine freie salzbildende Verbindung in ein Salz überführt.

Metallradikale sind beispielsweise Gruppen $-M^{I}$, $-M^{II}-Hal$ oder $-M^{II}/2$, worin $M^{I}$ ein Metallatom der Gruppe I, $M^{II}$ ein Metallatom der Gruppe II des Periodischen Systems der Elemente und Hal Halogen, wie Chlor, Brom oder Jod bedeutet. Bevorzugte Metallradikale der genannten Art sind solche der Formeln $-Li$, $-MgHal$ und $-Cd/2$.

Verbindungen der Formel V sind beispielsweise entsprechende Halogenameisensäureester, z. B. Chlorameisensäureniederalkylester. Halogenatome sind beispielsweise Chlor-, Brom- oder Jodatome.

Die Umsetzung von Verbindungen der Formeln IV und V erfolgt in üblicher Weise, vorteilhaft in einem inerten Lösungsmittel, wie einem Äther, z. B. in Diäthyläther oder Tetrahydrofuran, einem Kohlenwasserstoff, z. B. Benzol, oder Gemischen derselben, erforderlichenfalls unter Kühlen oder gelindem Erwärmen, z. B. bei etwa $-30°C$ bis etwa $100°C$, z. B. bei Siedetemperatur, und/oder unter Inertgas, z. B. unter Stickstoff. Bevorzugte Ausführungsformen dieses Verfahrens sind insbesondere die Umsetzung von Lithium- oder Halogenmagnesiumverbindungen der Formel IV mit Kohlendioxid, einem Halogen-, z. B. Chlor- oder Bromameisensäureniederalkylester oder einem Carbamylhalogenid, z. B. mit Carbamylchlorid oder N,N-Diniederalkylcarbamylchloriden.

Die Ausgangsstoffe der Formeln IV und V sind bekannt oder können, soweit neu, nach an sich bekannten Methoden hergestellt werden.

Die als Ausgangsstoffe zu verwendenden metallorganischen Verbindungen der Formel IV werden vorteilhaft in situ hergestellt, indem man eine entsprechende Halogen-, z. B. Chlor-, Jod- oder vor allem Bromverbindung, vorzugsweise in einem Äther, z. B. in Diäthyläther oder Tetrahydrofuran, mit Lithium oder vor allem Magnesium oder die entsprechende 2-unsubstituierte Verbindung in üblicher Weise, mit einer Kohlenwasserstoffmetallverbindung, wie Butyllithium, Butylmagnesiumbromid oder Phenyllithium, umsetzt. Andere metallorganische Verbindungen können aus den so erhältlichen Halogen- vor allem Brommagnesiumverbindungen, durch Umsetzung mit einem entsprechenden Metallhalogenid, z. B. mit Cadmiumchlorid, Kupferchlorid oder Zinkchlorid, erhalten werden. Die dafür zu verwendenden Halogenverbindungen können z. B. hergestellt werden, indem man ein entsprechendes, gegebenenfalls an einer Aminogruppe durch einen Rest $R_2$ substituiertes $R_1-X-1,2$-phenylendiamin, zugänglich z. B. aus dem entsprechenden Halogendinitrobenzol durch Reduktion der Nitrogruppen, z. B. mit Wasserstoff und Raney-Nickel, mit einem reaktionsfähigen Derivat der Kohlensäure, z. B. mit einem Diniederalkylcarbonat oder mit Phosgen umsetzt und die primär erhaltene 2-Hydroxyverbindung in üblicher Weise, z. B. mit Phosphortri- oder -pentachlorid oder Thionylchlorid, halogeniert. In analoger Weise können auch Ausgangsstoffen der Formel IV entsprechende 2-unsubstituierte Verbindungen hergestellt werden, indem man einen entsprechenden 1,2-Diaminohalogenbenzol, Aminonitro- oder $R_2$-Aminonitrohalogenbenzol mit Ameisensäure, oder einem Ester derselben umsetzt und gegebenenfalls die Nitrogruppe reduziert, wobei Ringschluß eintritt.

Die neuen Verbindungen, in denen $R_1-X-$ einen aliphatischen, cycloaliphatischen, cycloaliphatisch-aliphatischen, araliphatischen oder heterocyclisch-aliphatischen Rest bedeutet, können ferner hergestellt werden, indem man in einer entsprechenden Verbindung der Formel

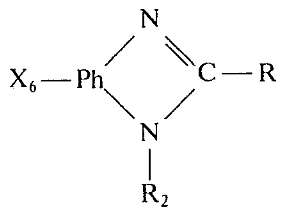

(V)

worin $X_6$ einen zu der Gruppe der Formel $R_1-X$ reduzierbaren Rest bedeutet, oder einem Salz davon die Gruppe $X_6$ zur gewünschten Gruppe der Formel $R_1-X-$ reduziert, und wenn erwünscht, eine so erhältliche Verbindung der Formel I in eine andere Verbindung der Formel I umwandelt, und/oder, wenn erwünscht, ein erhaltenes Salz in die freie Verbindung oder in ein anderes Salz und/oder eine freie salzbildende Verbindung in ein Salz überführt.

Zu den genannten Gruppen der Formel $R_1-X-$ reduzierbare Reste sind beispielsweise im aliphatischen und/oder cycloaliphatischen Teil mindestens eine Doppel- oder Dreifachbindung aufweisende oder durch mindestens einen reduktiv durch Wasserstoff ersetzbaren Rest $X_7$ substituierte aliphatische, cycloaliphatische, araliphatische, cycloaliphatisch-aliphatische oder heterocyclisch-aliphatische Reste. Mindestens eine Doppel- oder Dreifachbindung aufweisende Reste der genannten Art sind beispielsweise entsprechende Alkenyl- oder Alkinylreste, Cycloalkenyl-, Cycloalkenylalkyl- oder Cycloalkenylalkenylreste, Aralkylreste oder Heteroarylalkenylreste. Durch mindestens einen reduktiv durch Wasserstoff ersetzbaren Rest $X_7$, als welcher z. B. Hydroxy, Oxo und Halogen, wie Chlor, in Betracht kommt, substituierte Reste der genannten Art sind beispielsweise $X_7$-Alkylreste, $X_7$-Cycloalkylalkyl- oder Cycloalkyl-$X_7$-alkylreste, Aryl-$X_7$-alkylreste oder Heteroaryl-$X_7$arylreste, vorzugsweise der Formel $R_1-CH(X_7)-$, wie $R_1-CH(OH)-$.

Die Reduktion der Gruppe $X_6$, wobei eine Hydroxy $X_7$ aufweisende Gruppe $X_6$, z. B. der Formel $R_1-CH(OH)-$, auch in situ im Verlaufe der Oxidationsreaktion, z. B. aus der entsprechenden Oxo als Substituenten aufweisenden Gruppe, z. B. der Formel $R_1-C(=O)$, gebildet oder aus einem ihrer Derivate, wie Ester, z. B. Halogenwasserstoff- oder Niederalkansäureester, in Freiheit gesetzt werden kann, erfolgt in üblicher Weise. Als Reduktionsmittel kommt beispielsweise katalytisch erregter Wasserstoff, wie Wasserstoff in Gegenwart eines Hydrierungskatalysators, z. B. eines Platin-, Palladium- oder Nickelkatalysators, z. B. von Palladium auf Kohle, in Betracht. Dabei arbeitet man vorteilhafterweise in Gegenwart eines geeigneten Lösungs- oder Verdünnungsmittels, wie Äthanol, bei normaler Temperatur oder, wenn notwendig, unter Kühlen oder Erwärmen, z. B. in einem Temperaturbereich von etwa 0° C bis etwa 80° C.

Die als Ausgangsstoffe zu verwendenden Verbindungen der Formel VI können z. B. hergestellt werden, indem man in an sich bekannter Weise ein entsprechendes Chlorbenzol der Formel $H-PhH-Cl$ durch Umsetzung mit einer den Rest $X_6$ einführenden Verbindung, z. B. der Formel $R_1-COHal$ oder $(R_1CO)_2O$ in Gegenwart von Aluminiumtrichlorid acyliert, die so erhältliche Verbindung der Formel $X_6-PhH-Cl$ mit Salpetersäure/Schwefelsäure nitriert und die so erhältliche Chlornitroverbindung der Formel $X_6-Ph(Cl)-NO_2$, z. B. der Formel $R_1-CO-Ph(Cl)-NO_2$, mit Ammoniak oder einem Amin der Formel $R_2NH_2$ umsetzt, die so erhältliche Verbindung der Formel $X_6-Ph(NHR_2)-NO_2$ unter milden Bedingungen, z. B. mit Wasserstoff in Gegenwart von Palladium auf Kohle, vorteilhaft in einem inerten Lösungsmittel, wie Äthanol, gegebenenfalls in Gegenwart von Chlorwasserstoff und unter normalen Temperatur- und Druckbedingungen reduziert und die so erhältliche Verbindung $X_6-Ph(NHR_2)-NH_2$, z. B. der Formel $R_1-CH(OH)-Ph(NHR_2)-NH_2$, mit einer Säure der Formel $R-COOH$ oder einem geeigneten funktionellen Derivat davon, z. B. deren Niederalkylester kondensiert.

Eine erfindungsgemäß erhältliche Verbindung der Formel I kann in an sich bekannter Weise in eine andere Verbindung der Formel I umgewandelt werden.

So kann man in einer Verbindung der Formel I, worin R für Carboxy steht, dieses nach an sich bekannten Veresterungsverfahren in eine veresterte Carboxygruppe umwandeln. So kann man z. B. durch Behandeln mit einer geeigneten Diazoverbindung, wie einem Diazoniederalkan, mit einem geeigneten N,N-Diniederalkylformamidacetal, z. B. N,N-Dimethylformamiddiäthylacetal oder N,N-Dimethylformamid-methosulfat, oder einem Oxoniumsalz, wie mit einem Triniederalkyloxonium-tetrafluoroborat oder -hexafluorophosphat, mit einem Carbonat oder Pyrocarbonat, z. B. mit Diäthyl(pyro)carbonat, oder mit organischem Sulfit oder Phosphit, wie Diniederalkylsulfit oder Triniederalkylphosphit, in Gegenwart eines geeigneten sauren Mittels, wie p-Toluolsulfonsäure, oder mit einem Alkohol in Gegenwart eines geeigneten Kondensationsmittels, wie eines dehydratisierenden Mittels, z. B. Dicyclohexylcarbodiimid, oder, zur Bildung einer Hydroxyniederalkylgruppe, mit einem Epoxyniederalkan, z. B. Äthylenoxyd, verestern. Ferner kann man eine Verbindung der Formel I, worin eine freie Carboxylgruppe R in Salzform, z. B. in der Alkalimetall-, wie Natriumsalzform, vorliegt, mit einem reaktionsfähigen Ester eines Alkohols, z. B. mit einer starken Säure, wie einem entsprechenden Halogenid, z. B. Chlorid, Bromid oder Jodid, oder Schwefelsäureester, oder eine Verbindung der Formel I, worin eine freie Carboxylgruppe R in einer Anhydridform, vorzugsweise als

Halogencarbonyl-, z. B. Chlorcarbonylgruppe, vorliegt, die man z. B. durch Behandeln einer Verbindung der Formel I, worin R für Carboxy steht, mit einem Halogenierungsmittel, z. B. Thionylchlorid, bilden kann, mit einem Metallalkoholat oder einem Alkohol in Gegenwart einer säurebindenden Base umsetzen, und so zu einer Verbindung der Formel I gelangen, worin R für verestertes Carboxy steht. Dabei können in einem Veresterungsreagens gegebenenfalls vorhandene Substituenten in funktionell abgewandelter Form vorliegen und dann in einer Verbindung der Formel I, worin R z. B. für substituiertes Niederalkoxycarbonyl steht, in welchem Substituenten in funktionell abgewandelter Form vorliegen, freigesetzt werden. So kann man als Veresterungsreagens z. B. das 2,3-Epoxy-propylchlorid verwenden und im erhaltenen Ester eine 2,3-Epoxy-propyloxygruppierung R nachträglich zur gewünschten 2,3-Dihydroxy-propyloxygruppierung hydrolysieren.

In einer Verbindung der Formel I, worin R für verestertes Carboxy, z. B. auch p-Nitro- bzw. 2,4-Dinitrophenoxy- oder -benzyloxycarbonyl steht, kann dieses durch Umesterung, z. B. durch Behandeln mit einem Alkohol, erforderlichenfalls in Gegenwart eines geeigneten Umesterungskatalysators, wie eines gegebenenfalls substituierten Alkalimetall-, z. B. Natrium- oder Kaliumalkanolats, in eine andere veresterte Carboxygruppe umgewandelt werden.

In einer erhaltenen Verbindung der Formel I, worin R für freies, anhydridisiertes oder verestertes Carboxy steht, kann dieses in an sich bekannter Weise ferner in gegebenenfalls substituiertes Carbamyl umgewandelt werden. So kann man eine Verbindung der Formel I, worin eine Carboxylgruppe R in einer Anhydridform, insbesondere als Halogencarbonyl-, z. B. Chlorcarbonylgruppe, oder in veresterter Form vorliegt, mit Ammoniak, Hydroxylamin oder einem primären oder sekundären Amin behandeln und so zu Verbindungen der Formel I gelangen, worin R für gegebenenfalls substituiertes Carbamyl steht. Ferner kann man das Ammoniumsalz oder ein Aminsalz einer Verbindung der Formel I, worin R für Carboxy steht, durch Dehydratisieren mit einem geeigneten Dehydratisierungsmittel, wie Schwefelsäure, in eine Verbindung der Formel I überführen, worin R gegebenenfalls substituiertes Carbamyl bedeutet.

Die erwähnten Verbindungen, in denen R in Halogenidform vorliegendes Carboxy ist, können ausgehend von Verbindungen der Formel I, worin R für Carboxy steht durch Behandeln mit einem Thionylhalogenid, wie Thionylchlorid hergestellt werden. Ist $R_2$ Wasserstoff, können diese zu Verbindungen der Formel

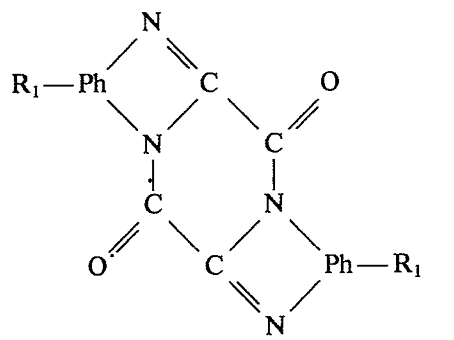

dimerisieren. Ein solches Zwischenprodukt kann z. B. durch Behandeln mit einem geeigneten Alkoholat, wie einem Alkalimetall-, z. B. Natrium- oder Kaliumalkoholat, oder mit einem Alkohol in Gegenwart einer Mineralsäure, z. B. Chlorwasserstoff, oder mit Ammoniak, Hydroxylamin oder einem primären oder sekundären Amin, in eine Verbindung der Formel I übergeführt werden, worin R für verestertes Carboxy bzw. gegebenenfalls substituiertes Carbamyl steht.

In einer Verbindung der Formel I kann man eine veresterte Carboxylgruppe oder eine gegebenenfalls substituierte Carbamylgruppe R in üblicher Weise in die freie Carboxylgruppe überführen, z. B. durch Hydrolyse, normalerweise in einem alkalischen Medium, wie durch Behandeln mit Wasser in Gegenwart eines Alkalimetall- oder Erdalkalimetallhydroxids, z. B. Natriumhydroxid.

In einer Verbindung der Formel I, worin $R_2$ für Wasserstoff steht, kann dieses, z. B. durch Behandeln mit einem reaktionsfähigen Ester eines entsprechenden Alkohols, wie einem Halogenid, in Gegenwart einer Base, z. B. eines Alkalimetallalkoholats, durch einen aliphatischen Rest ersetzt werden.

Eine gegebenenfalls veresterte oder in Halogenid- oder Salzform vorliegende Carboxygruppe R kann ferner durch Umsetzung mit einem Leichtmetallborhydrid oder mit Wasserstoff in Gegenwart eines Hydrierungskatalysators zu Hydroxymethyl reduziert werden. Zur Reduktion einer gegebenenfalls veresterten oder als Alkalimetall-, wie Natriumsalz, vorliegenden Carboxygruppe verwendet man vorzugsweise ein Leichtmetallhydrid, wie ein Boran, z. B. Diboran oder den Borantetrahydrofurankomplex oder ein Dileichtmetallhydrid, wie Lithiumaluminiumhydrid, Natriumborhydrid oder Natriumcyanoborhydrid. Halogencarbonylgruppen wie Chlorcarbonyl, reduziert man vorzugsweise mit Wasserstoff in Gegenwart von Palladium, vorzugsweise auf einem Träger, wie Bariumsulfat, und erforderlichenfalls eines schwefelhaltigen Cokatalysators, z. B. von Thioharnstoff.

Ferner kann man in einer Verbindung der Formel I, worin R für Hydroxymethyl steht, dieses in

üblicher Weise, z. B. durch Umsetzung mit einem veräthernden Mittel, in eine verätherte Hydroxymethylgruppe überführen. Veräthernde Mittel sind beispielsweise reaktive Ester entsprechender Alkohole, beispielsweise deren Ester mit anorganischen Säuren, wie Chlor-, Brom- oder Jodwasserstoffsäure oder Schwefelsäure, oder mit organischen Sulfonsäuren, z. B. mit Methan-, Benzol-, p-Brombenzol- oder p-Toluolsulfonsäure, ferner von entsprechenden 1,2-Diolen abgeleitete Epoxide. Die Umsetzung mit den genannten veräthernden Mitteln kann in üblicher Weise erfolgen, beispielsweise in Gegenwart eines Alkalimetallhydrides oder -alkoholates, z. B. von Natriumhydrid oder Natriummethanolat, oder indem man die zu veräthernde Verbindung als Salz, z. B. Natriumsalz, einsetzt. Ferner kann man in einer Verbindung der Formel I, in der R Hydroxymethyl ist, dieses in üblicher Weise verestern, z. B. durch direkte Veresterung mit einer entsprechenden Carbonsäure in Gegenwart einer Mineralsäure, z. B. von Chlorwasserstoffsäure oder Schwefelsäure, oder durch Umsetzung mit einem reaktiven Derivat, z. B. einem Anhydrid, wie dem Anhydrid oder Chlorid, oder einem Ester, wie Niederalkyl- oder o-Nitrophenyl-, 2,4-Dinitrophenylester, der Carbonsäure, erforderlichenfalls in Gegenwart eines sauren oder vor allem basischen Kondensationsmittels, bei der Umsetzung mit einem Säureanhydrid, z. B. von Pyridin, und bei der Umsetzung mit einem Ester, z. B. eines Alkalimetall-, wie Natrium- oder Kaliumalkoholates, in eine veresterte Hydroxymethylgruppe R überführen. Die Verätherung bzw. Veresterung einer Hydroxymethylgruppe kann aber auch so durchgeführt werden, daß man diese zunächst in üblicher Weise, z. B. mit Phosphortribromid oder Thionylchlorid in eine Halogenmethylgruppe überführt und anschließend mit einem Alkalimetall-, z. B. dem Natriumalkoholat, des entsprechenden Alkohols, oder einem Alkalimetall-, z. B. dem Natriumsalz der entsprechenden Carbonsäure umsetzt.

Gegebenenfalls veresterte Hydroxymethylgruppen R kann man weiterhin zu Carboxygruppen und verätherte Hydroxymethylgruppen zu veresterten Carboxygruppen oxydieren. Die Oxydation kann in an sich bekannter Weise, z. B. durch Umsetzung mit einer oxydierenden Schwermetallverbindung, ausgehend von Hydroxymethyl vorzugsweise mit einer Chrom-VI- oder Mangan-VII- enthaltenden oxydierenden Verbindung, z. B. mit Chromtrioxid oder insbesondere Kaliumpermanganat, ausgehend von veräthertem Hydroxymethyl R ferner mit einer Mangan-IV- enthaltenden Verbindung, wie Mangandioxid, durchgeführt werden. Dabei arbeitet man vorzugsweise in Gegenwart eines geeigneten Lösungs- oder Verdünnungsmittels, z. B. von Aceton oder Pyridin, oder eines vorzugsweise wäßrigen, Gemisches davon, wenn notwendig unter Kühlen oder Erwärmen, z. B. in einem Temperaturenbereich von etwa 0° C bis etwa 80° C.

Erhaltene freie salzbildende Verbindungen der Formel I können in an sich bekannter Weise in Salze übergeführt werden, Säuren z. B. mit einer Base oder mit einem geeigneten Salz einer Carbonsäure und Basen mit einer Mineralsäure, üblicherweise in Gegenwart eines Lösungs- oder Verdünnungsmittels.

Erhaltene Salze können in an sich bekannter Weise in die freien Verbindungen umgewandelt werden, z. B. durch Behandeln mit einem sauren Reagens, wie einer Mineralsäure.

Die Verbindungen, inklusive ihre Salze können auch in Form ihrer Hydrate erhalten werden oder das zur Kristallisation verwendete Lösungsmittel einschließen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckgemäß gegebenenfalls auch die entsprechenden Salze bzw. freien Verbindungen zu verstehen.

Die Erfindung betrifft auch diejenigen Ausführungsformen des Verfahrens, bei denen man von einer auf beliebiger Verfahrensstufe als Zwischenprodukt erhältlichen Verbindung ausgeht und die fehlenden Verfahrensschritte ausführt, oder einen Ausgangsstoff unter den Reaktionsbedingungen gebildet oder in Form eines Derivates davon, gegebenenfalls eines Salzes, verwendet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu ihrer Herstellung bilden ebenfalls einen Gegenstand der vorliegenden Erfindung.

Die vorliegende Erfindung betrifft ebenfalls pharmazeutische Präparate, welche Verbindungen der Formel I oder pharmazeutisch verwendbare Salze davon enthalten. Bei den erfindungsgemäßen pharmazeutischen Präparaten handelt es sich um solche zur enteralen, wie oralen, nasalen oder rektalen, sowie parenteralen oder topischen Verabreichung an Warmblüter, welche den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab.

Die neuen pharmazeutischen Präparate enthalten z. B. bis etwa 95%, vorzugsweise von etwa 5% bis etwa 90% des Wirkstoffs. Erfindungsgemäße pharmazeutische Präparate sind z. B. solche in Dosiseinheitsformen, wie Dragées, Tabletten, Kapseln oder Suppositorien, sowie Ampullen, ferner Inhalationspräparate, ferner topisch und lokal (z. B. zur Insufflation) verwendbare pharmazeutische Zubereitungen.

Die pharmazeutischen Präparate der vorliegenden Erfindung werden in an sich bekannter Weise, z. B. mittels konventioneller Misch-, Granulier-, Dragier-, Lösungs- oder Lyophilisierungsverfahren

hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z. B. Lactose, Saccharose, Mannit oder Sorbit, Cellulosepräparate und/oder Calciumphosphate, z. B. Tricalciumphosphat oder Calciumhydrogenphosphat, ferner Bindemittel, wie Stärkekleister unter Verwendung z. B. von Mais-, Weizen-, Reis- oder Kartoffelstärke, Gelatine, Traganth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fließregulier- und Schmiermittel, z. B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Überzügen versehen, wobei man u. a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z. B. zur Identifizierung oder zur Kennzeichnung verschiedener Wirkstoffdosen, beigefügt werden.

Weitere, oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z. B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Ölen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z. B. Suppositorien in Betracht, welche aus einer Kombination des Wirkstoffs mit einer Suppositoriengrundmasse bestehen. Als Suppositoriengrundmasse eignen sich z. B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z. B. flüssige Triglyceride, Polyäthylenglykole oder Paraffinkohlenwasserstoffe in Frage.

Zur parenteralen Verabreichung eignen sich in erster Linie wäßrige Lösungen eines Wirkstoffs in wasserlöslicher Form, z. B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z. B. Sesamöl, oder synthetische Fettsäureester, z. B. Äthyloleat oder Triglyceride, verwendet, oder wäßrige Injektionssuspensionen, welche viskositätserhöhende Stoffe, z. B. Natriumcarboxymethylcellulose, Sorbit und/oder Dextran und gegebenenfalls auch Stabilisatoren enthalten.

Inhalationspräparate für die Behandlung der Atemwege durch nasale oder buccale Verabreichung sind z. B. Ärosole oder Sprays, welche den pharmakologischen Wirkstoff in Form eines Puders oder in Form von Tropfen einer Lösung oder Suspension verteilen können. Präparate mit Puder-verteilenden Eigenschaften enthalten außer dem Wirkstoff üblicherweise ein flüssiges Treibgas mit einem Siedepunkt unter der Raumtemperatur, sowie, wenn erwünscht, Trägerstoffe, wie flüssige oder feste nicht-ionische oder anionische oberflächenaktive Mittel und/oder feste Verdünnungsmittel. Präparate, in welchen der pharmakologische Wirkstoff in Lösung vorliegt, enthalten außer diesem ein geeignetes Treibmittel, ferner, falls notwendig, ein zusätzliches Lösungsmittel und/oder einen Stabilisator. Anstelle des Treibgases kann auch Druckluft verwendet werden, wobei diese mittels einer geeigneten Verdichtungs- und Entspannungsvorrichtung nach Bedarf erzeugt werden kann.

Pharmazeutische Präparate für topische und lokale Verwendung sind z. B. für die Behandlung der Haut Lotionen und Cremen, die eine flüssige oder semifeste Öl-in-Wasser- oder Wasser-in-Öl-Emulsion enthalten, und Salben (wobei solche vorzugsweise ein Konservierungsmittel enthalten), für die Behandlung der Augen Augentropfen, welche die aktive Verbindung in wäßriger oder öliger Lösung enthalten und Augensalben, die vorzugsweise in steriler Form hergestellt werden, für die Behandlung der Nase Puder, Aerosole und Sprays (ähnlich den oben beschriebenen für die Behandlung der Atemwege), sowie grobe Puder, die durch schnelles Inhalieren durch die Nasenlöcher verabreicht werden, und Nasentropfen, welche die aktive Verbindung in wäßriger oder öliger Lösung enthalten, oder für die lokale Behandlung des Mundes Lutschbonbons, welche die aktive Verbindung in einer im allgemeinen aus Zucker und Gummiarabikum oder Tragakanth gebildeten Masse enthalten, welcher Geschmacksstoffe beigegeben sein können, sowie Pastillen, die den Aktivstoff in einer inerten Masse, z. B. aus Gelatine und Glycerin oder Zucker und Gummiarabikum, enthalten.

Die Erfindung betrifft ebenfalls die Verwendung der neuen Verbindungen der Formel I oder Salzen davon als pharmakologisch aktive Verbindungen, insbesondere als Antiallergika, vorzugsweise in der

Form von pharmazeutischen Präparaten. Die tägliche Dosis, die einem Warmblüter von etwa 70 kg verabreicht wird, beträgt je nach Applikationsform, von etwa 2 mg bis etwa 7000 mg.

Die nachfolgenden Beispiele illustrieren die oben beschriebene Erfindung; sie sollen jedoch diese in ihrem Umfang in keiner Weise einschränken. Temperaturen werden in Celsiusgraden angegeben.

## Beispiel 1

23 g rohes 4-Butyl-1,2-phenylen-diamin werden mit 20,8 g Äthoxyessigsäure versetzt und 90 Minuten auf 130° erhitzt. Man läßt abkühlen, nimmt mit Essigsäureäthylester auf, wäscht mit Natriumhydrogencarbonatlösung und anschließend dreimal mit Wasser, trocknet über Natriumsulfat, filtriert und dampft unter vermindertem Druck zur Trockne ein. Der Rückstand wird an 600 g Kieselgel mit Chloroform als Laufmittel chromatographiert. Nach wenig Vorlauf erhält man in der Hauptfraktion das 2-Äthoxymethyl-5-butyl-benzimidazol.

Das Ausgangsmaterial kann folgendermaßen hergestellt werden:

Eine Lösung von 18,3 g 4-Chlor-butyrophenon in 100 ml Schwefelsäure von −20° wird innerhalb von 5 Minuten bei −20 bis −15° mit einer Mischung von 40 ml Schwefelsäure und 21 ml rauchender Salpetersäure versetzt, wobei alles in Lösung geht. Man rührt bei −15° bis −10° 45 Minuten nach, gießt auf 1000 g Eis, saugt ab, wäscht mit Wasser nach, nimmt in Chloroform auf, wäscht mit gesättigter Natriumbicarbonatlösung und zweimal mit Wasser, trocknet über Natriumsulfat, filtriert und dampft zur Trockne ein. Der Eindampfrückstand wird mit 25 ml Methanol digeriert. Man erhält das 4-Chlor-3-nitro-butyrophenon vom Smp. 52−54°.

Auf eine Lösung von 22,8 g 4-Chlor-3-nitro-butyrophenon in 300 ml Äthanol werden im Autoklaven 50 g Ammoniak aufgepreßt. Es wird 10 Stunden auf 100° erwärmt, nach dem Abkühlen auf Raumtemperatur unter vermindertem Druck zur Trockne eingedampft, mit 200 ml 2n-Salzsäure 1 Stunde auf 80−90° erwärmt, durch Zugabe von Eis auf 15° abgekühlt, abgesaugt und mit Wasser nachgewaschen. Das Nutschgut wird in 1000 ml Methylenchlorid aufgenommen, über Natriumsulfat getrocknet, eingeengt, mit Petroläther (Siedebereich 60−80°) versetzt und das Methylenchlorid vollends abgedampft. Das kristallin ausgefallene 4-Amino-3-nitro-butyrophenon wird abgesaugt und im Vakuum getrocknet. Es schmilzt bei 128−129°.

19,9 g 4-Amino-3-nitro-butyrophenon werden in etwa 300 ml Äthanol und 15 ml 12,8%iger äthanolischer Salzsäure gelöst und nach Zugabe von Palladium 5%ig auf Kohle (2 g) bei etwa 30−35° bis zur Aufnahme von 11,2 l Wasserstoff hydriert. Der Katalysator wird abfiltriert und das Filtrat unter vermindertem Druck eingedampft. Der Rückstand wird in Äther aufgenommen, über Natriumsulfat getrocknet und eingedampft. Man erhält das 4-Butyl-1,2-phenylendiamin, das ohne weitere Reinigung weiterumsetzt werden kann.

## Beispiel 2

4,65 g 2-Äthoxymethyl-5-butyl-benzimidazol werden in 100 ml Aceton und 5 ml Wasser gelöst, mit 5 g Kaliumpermanganat versetzt und 6 Stunden zum Rückfluß erhitzt, wobei in etwa halbstündigen Abständen je 1 g Kaliumpermangant (insgesamt 10 g) zugegeben werden. Dann wird heiß filtriert und unter vermindertem Druck eingedampft. Der Rückstand wird mit Essigester aufgenommen, mit Eis versetzt und mit schwach angesäuerter Natriumbisulfitlösung ausgeschüttelt. Die wäßrige Phase wird abgetrennt und zweimal mit Essigester ausgeschüttelt. Die organischen Phasen werden vereinigt, zweimal mit Wasser gewaschen, über Natriumsulfat getrocknet und eingedampft. Man erhält den 5-Butyl-benzimidazol-2-carbonsäureäthylester in Form eines gelblichen Öls, welches nach einigen Stehen spontan kristallisiert (Smp. 129−130°).

## Beispiel 3

25 g 5-Butyl-6-methyl-2-methylaminoanilin-bishydrochlorid werden in 150 ml 2n Salzsäure gelöst, mit 15,6 g Äthoxyessigsäure versetzt und 5 Stunden zum Rückfluß erhitzt. Man läßt abkühlen, versetzt mit Eis, macht mit konzentrierter Natronlauge alkalisch und extrahiert dreimal mit Essigsäureäthylester. Der Auszug wird zweimal mit Wasser gewaschen, über Natriumsulfat getrocknet und unter vermindertem Druck zur Trockne eingedampft. Man erhält das 2-Äthoxymethyl-5-butyl-1,6-dimethylbenzimidazol.

Das Ausgangsmaterial kann wie folgt erhalten werden:

Die gelbe Suspension von 900 ml 3-Chlor-toluol und 367,5 g Aluminiumchlorid (fein pulverisiert) wird innerhalb einer Stunde mit 266 g Buttersäurechlorid versetzt. Während des Zutropfens entwickelt sich Chlorwasserstoffgas; die Reaktion ist exotherm (man läßt die Temperatur auf 70° steigen) und das Aluminiumchlorid löst sich auf. Nach Beendigung der Zugabe des Buttersäurechlorids wird das Reaktionsgemisch bis zum Aufhöhren der Gasentwicklung bei 70° gehalten (etwa 45 Minuten), kühlt dann auf 50° und gießt auf 2500 g Eis aus.

Je zwei gleiche Ansätze werden zusammengenommen und mit Essigsäureäthylester extrahiert; der organische Extrakt wird zweimal mit 2-n. Salzsäure, einmal mit einer gesättigten wäßrigen

8000 ml Eiswasser aus; das ausgefallene Öl wird mit Chloroform extrahiert. Der organische Extrakt wird einmal mit einer wäßrigen Natriumhydrogencarbonatlösung und einmal mit Wasser gewaschen, über Natriumsulfat getrocknet und eingedampft. Der Rückstand wird in der doppelten Menge heißem Methanol gelöst und während 16 Stunden stehengelassen. Der kristalline Niederschlag wird abfiltriert, mit kaltem Wasser gewaschen und bei 100 mm Hg und Raumtemperatur während 18 Stunden getrocknet. Man erhält so das 4-Chlor-2-methyl-5-nitro-butyrophenon, das bei 71 – 72° schmilzt.

Ein Gemisch von 24,1 g 4-Chlor-2-methyl-5-nitro-butyrophenon und 250 ml einer 33%igen Lösung von Methylamin in Äthanol wird bei Raumtemperatur stehengelassen; das kristalline Ausgangsmaterial löst sich langsam auf, wobei Gelbfärbung eintritt. Die Reaktion ist schwach-exotherm; man kühlt deshalb mit einem Wasserbad, um ein zu starkes Entweichen von Methylamin zu verhindern. Nach 20 Minuten tritt vollständige Lösung ein, dann beginnt ein Niederschlag auszufallen. Man läßt während 16 Stunden bei Raumtemperatur stehen und dampft dann unter vermindertem Druck zur Trockne ein. Der Rückstand wird mit Diäthyläther (etwa 1000 ml), Eis und Natriumcarbonat versetzt, durchgeschüttelt und die organische Schicht abgetrennt. Diese wird zweimal mit Wasser gewaschen und die wäßrige Lösung mit Diäthyläther zurückgewaschen. Die vereinigten organischen Lösungen werden über Natriumsulfat getrocknet, filtriert und auf ein Volumen von etwa 300 ml eingedampft, dann mit 100 ml Petroläther verdünnt und gekühlt. Das gelbe, kristalline 2-Methyl-4-methylamino-5-nitro-butyrophenon fällt aus, wird abfiltriert, mit Petroläther gewaschen und an der Luft getrocknet, F. 107 – 108°.

Die Überführung von 4-Chlor-2-methyl-5-nitro-butyrophenon in 4-Amino-2-methyl-5-methylamino-butyrophenon kann auch folgendermaßen durchgeführt werden, wobei man auch von einem rohen Isomerengemisch ausgehen kann.

241 g rohes Chlor-methyl-nitrobutyrophenon (etwa 75%ig an 4-Chlor-2-methyl-5-nitro-butyrophenon) werden in 1200 ml Äthanol suspendiert und mit 1200 ml 33%iger Methylaminlösung versetzt, worauf unter exothermer Reaktion Auflösung erfolgt. Man läßt 2 Tage stehen, dampft unter vermindertem Druck zur Trockne ein, versetzt mit 600 ml 2n-Salzsäure und erwärmt 1 Stunde auf 80 – 90°. Man kühlt durch Zugabe von Eis auf etwa 15° ab, saugt den kristallinen Niederschlag ab, wäscht mit Wasser nach, nimmt in Methylenchlorid auf, trocknet über Natriumsulfat, dampft unter verminderten Druck, zuletzt unter Zugabe von Cyclohexan und Petroläther (Siedebereich 60 – 80°), das Methylenchlorid ab, kühlt und saugt das 2-Methyl-4-methylamino-5-nitrobutyrophenon vom Smp. 105 – 107° ab.

23,6 g 2-Methyl-4-methylamino-5-nitro-butyrophenon werden in 240 ml Äthanol und 57 ml 12,8%iger äthanolischer Salzsäure gelöst, mit 5%iger Palladiumkohle (7,4 g) versetzt und bei 30 – 35° bis zur Aufnahme von 11,6 Liter Wasserstoff hydriert. Man filtriert vom Katalysator ab, dampft unter vermindertem Druck zur Trockne ein, suspendiert in Toluol, destilliert das Wasser azeotrop ab und filtriert die Kristalle des 4-Butyl-6-methyl-2-methylamino-anilin-bis-hydrochlorid vom Smp. über 160° ab.

## Beispiel 4

27,0 g rohes 2-Äthoxymethyl-5-butyl-1,6-dimethylbenzimidazol werden in analoger Weise wie in Beispiel 2 beschrieben mit 50 g Kaliumpermanganat zum 5-Butyl-1,6-dimethylbenzimidazol-2-carbonsäureäthylester vom Smp. 56 – 57° oxydiert.

## Beispiel 5

45 g rohes 4-Butyl-2-methylamino-anilin-bishydrochlorid werden in 200 ml 2n-Salzsäure gelöst, mit 23,4 g Äthoxyessigsäure versetzt und 2 Stunden zum Rückfluß erhitzt. Man läßt abkühlen, fügt bis zur deutlich alkalischem Reaktion konzentrierte Natronlauge hinzu und extrahiert dreimal mit Essigsäureäthylester. Die Auszüge werden vereinigt, zweimal mit Wasser gewaschen, über Natriumsulfat getrocknet, unter vermindertem Druck eingedampft. Der Eindampfrückstand wird aus wenig Petroläther umkristallisiert. Man erhält das 2-Äthoxymethyl-5-butyl-1-methyl-benzimidazol vom Smp. 55 – 58°.

Das Ausgangsmaterial kann in analoger Weise wie in Beispiel 3 beschrieben ausgehend von Chlorbenzol über 4-Chlorbutyrophenon und 4-Chlor-3-nitrobutyrophenon hergestellt werden.

## Beispiel 6

8,9 g 2-Äthoxymethyl-5-butyl-1-methyl-benzimidazol werden in 180 ml Aceton und 9 ml Wasser gelöst, mit 10 g Kaliumpermanganat versetzt und 2 Stunden bei Raumtemperatur gerührt. Dann wird etwa 7 Stunden zum Rückfluß erhitzt, wobei in etwa halbstündigen Abständen jeweils 2 g Kaliumpermanganat zugegeben wird (insgesamt 22 g). Man läßt abkühlen, filtriert über Diatomeenerde, dampft unter vermindertem Druck zur Trockne ein und nimmt in Essigsäureäthylester

Natriumchloridlösung, zweimal mit einer 2-n. wäßrigen Natriumcarbonatlösung und einmal mit einer gesättigten wäßrigen Natriumchloridlösung gewaschen, getrocknet und eingedampft. Der so erhältliche braune, ölige Rückstand wird destilliert; man erhält das Gemisch des 4-Chlor-2-methyl-butyrophenons und des 2-Chlor-4-methyl-butyrophenons bei 160–164°/14 mm Hg.

Konzentrierte Schwefelsäure (1275 ml), mittels eines Kohlendioxid/Chloroform-Gemisches auf –20° bis –25° gekühlt, wird unter gutem Rühren innerhalb von 10 Minuten tropfenweise mit 285,5 g des Gemisches von 4-Chlor-2-methyl-butyrophenon und 2-Chlor-4-methyl-butyrophenon versetzt. Die entstandene Lösung wird bei –20° bis –25° innerhalb von 30 Minuten mit einem Gemisch von 240 ml konzentrierter Schwefelsäure und 75 ml 100%iger Salpetersäure (d: 1,52) behandelt und anschließend während 15 Minuten weitergerührt, wobei man die Temperatur auf –15° erhöhen läßt. Man gießt auf auf. Die Lösung wird mit Natriumbisulfitlösung ausgeschüttelt, zweimal mit Wasser gewaschen, über Natriumsulfat getrocknet und unter vermindertem Druck eingedampft. Der Eindampfrückstand wird an Kieselgel mit einem Gemisch aus gleichen Teilen Chloroform, Petroläther und Essigsäureäthylester als Laufmittel chromatographiert. Man erhält den 5-Butyl-1-methyl-benzimidazol-2-carbonsäureäthylester vom Smp. 49–50°.

Beispiel 7

7 g 5-Butyryl-6-methyl-benzimidazol-2-carbonsäureäthylester werden in 140 ml Äthanol gelöst und nach Zugabe von 5%iger Palladiumkohle (3,5 g) bei 20–50° hydriert. Man filtriert vom Katalysator ab, dampft zur Trockne ein und chromatographiert an Kieselgel mit Methylenchlorid/Essigsäureäthylester (1 : 1) als Laufmittel. Man erhält den 5-Butyl-6-methyl-benzimidazol-2-carbonsäureäthylester vom Smp. 123–125°.

Das Ausgangsmaterial kann folgendermaßen hergestellt werden:

22,2 g 4-Amino-2-methyl-5-nitro-butyrophenon werden in 230 ml Methanol gelöst, mit 2 g Raney-Nickel versetzt und bei 15–25° bei Normaldruck bis zur Aufnahme von 4,9 Liter Wasserstoff hydriert. Man gibt unter Stückstoff 20,8 g Äthoxyessigsäure hinzu, filtriert vom Katalysator ab, dampft unter vermindertem Druck ein und erhitzt 3 Stunden auf 130°. Nach dem Abkühlen löst man in 200 ml 2n-Salzsäure, wäscht zweimal mit Essigsäureäthylester, macht in der Kälte mit Natriumcarbonat alkalisch und extrahiert zweimal mit Essigsäureäthylester, trocknet über Natriumsulfat, dampft ein und chromatographiert an 300 g Silicagel. Man eluiert zunächst einen Vorlauf mit 1200 ml Chloroform und dann mit 1200 ml Chloroform/Äthanol (24 : 1) das 2-Äthoxymethyl-5-butyryl-6-methyl-benzimidazol.

Zu einer auf 10° gekühlten Lösung von 18,9 g 2-Äthoxymethyl-5-butyryl-6-methyl-benzimidazol in 380 ml Aceton, 9,5 ml Pyridin und 5,7 ml Wasser werden unter Rühren 15 g Kaliumpermanganat hinzugefügt. Man läßt 1 Stunde unter Eiskühlung und 40 Stunden bei Raumtemperatur nachrühren, filtriert, dampft im Unterdruck zur Trockne ein, nimmt mit Essigsäureäthylester auf, wäscht nacheinander mit auf $P_H = 6$ gepufferter Natriumhydrogencarbonatlösung und zweimal mit Wasser, trocknet über Natriumsulfat und dampft unter vermindertem Druck ein. Dann nimmt man in 30 ml warmen Essigester auf, läßt über Nacht stehen und saugt den kristallinen 5-Butyryl-6-methyl-benzimidazol-2-carbonsäureäthylester vom Smp. 137–139° ab. Aus der Mutterlauge kann weiteres Produkt vom Smp. 129–132° gewonnen werden. Umkristallisieren aus Essigsäureäthylester/Methylenchlorid erhöht den Smp. 146–147°.

Beispiel 8

In analoger Weise wie in Beispiel 1 beschrieben erhält man durch Umsetzung von 25 g 5-Butyl-6-methyl-2-methylamino-anilin-bis-hydrochlorid mit 11,5 g Glykolsäure das 2-Hydroxymethyl-5-butyl-1,6-dimethyl-benzimidazol in Form eines viskosen Öls.

Beispiel 9

In analoger Weise wie in Beispiel 2 beschrieben erhält man durch Oxydation von 8,5 g 2-Hydroxymethyl-5-butyl-1,6-dimethyl-benzimidazol mit 24 g Kaliumpermanganat in 200 ml 95%igem wäßrigen Aceton die 5-Butyl-1,6-dimethyl-benzimidazol-2-carbonsäure.

Beispiel 10

Eine zur Inhalation geeignete, 2%ige wäßrige Lösung des Natriumsalzes der 5(6)-Methyl-benzimidazol-2-carbonsäure kann wie folgt hergestellt werden:

Zusammensetzung: (für 100 ml)

| | |
|---|---|
| Natriumsalz der 5(6)-Methyl-benzimidazol-2-carbonsäure | 2000 g |
| Dinatriumsalz der Äthylendiamintetraessigsäure (Stabilisator) | 0,010 g |

| | | |
|---|---|---|
| Benzalkoniumchlorid (Konservierungsmittel) | | 0,010 g |
| Wasser, destilliert | ad | 100 ml |

Das Natriumsalz der 5(6)-Methyl-benzimidazol-2-carbonsäure wird in frisch destilliertem Wasser gelöst, und die Lösung mit dem Dinatriumsalz der Äthylendiamin-tetraessigsäure und dem Benzalkoniumchlorid (Gemisch von Alkyl-dimethyl-benzyl-ammoniumchloriden, worin Alkyl von 8 bis 18 Kohlenstoffatome enthält) versetzt. Nach vollständiger Auflösung der Komponenten wird die erhaltene Lösung mit Wasser auf ein Volumen von 100 ml gebracht, abgefüllt und gasdicht verschlossen.

Analog können 2%ige wäßrige Inhalationslösungen des Natriumsalzes von 5,6-Dimethyl-benzimidazol-2-carbonsäure oder 5-Butyl-1,6-dimethyl-benzimidazol-2-carbonsäure hergestellt werden.

Beispiel 11

Zur Insufflation geeignete 0,025 g der 5-Butyl-1,6-dimethyl-benzimidazol-2-carbonsäureäthylester enthaltende Kapseln können wie folgt hergestellt werden:

Zusammensetzung: (für 1000 Kapseln)

| | |
|---|---|
| 5-Butyl-1,6-dimethyl-benzimidazol-2-carbonsäureäthylester | 25,00 g |
| Lactose, gemahlen | 25,00 g |

Die 5-Butyl-1,6-dimethyl-benzimidazol-2-carbonsäureäthylester und die Lactose (feinst gemahlen) werden gut miteinander vermischt. Das erhaltene Pulver wird dann gesiebt und die Portionen zu je 0,05 g in Gelatinekapseln abgefüllt.

In analoger Weise kann man auch Insufflationskapseln enthaltend je 0,025 g
2-Äthoxymethyl-5-butyl-1-methyl-benzimidazol,
2-Äthoxymethyl-5-butyl-1,6-dimethyl-benzimidazol,
2-Äthoxymethyl-5-butyl-benzimidazol,
5-Butyl-benzimidazol-2-carbonsäureäthylester,
5-Butyl-1-methyl-benzimidazol-2-carbonsäureäthylester und
5-Butyl-6-methyl-benzimidazol-2-carbonsäureäthylester
herstellen.

Beispiel 12

Tabletten enthaltend 100 mg Butyl-1,6-dimethyl-benzimidazol-2-carbonsäureäthylester (Wirkstoff) können beispielsweise in folgender Zusammensetzung hergestellt werden:

| Zusammensetzung | Pro Tablette |
|---|---|
| 5-Butyl-1,6-dimethyl-benzimidazol-2-carbonsäureäthylester | 100 mg |
| Milchzucker | 50 mg |
| Weizenstärke | 73 mg |
| Kolloidale Kieselsäure | 13 mg |
| Talk | 12 mg |
| Magnesiumstearat | 2 mg |
| | 250 mg |

Herstellung

Der Wirkstoff wird mit dem Milchzucker, einem Teil der Weizenstärke und mit kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist. Die Masse wird durch ein Sieb von ca. 3 mm Maschenweite getrieben, getrocknet und das trockene Granulat nochmals durch ein Sieb getrieben. Darauf werden die restliche Weizenstärke, Talk und Magnesiumstearat

zugemischt. Die erhaltene Mischung wird zu Tabletten von 250 mg mit Bruchkerbe(n) verpreßt.

In analoger Weise können auch Tabletten enthaltend jeweils 100 mg einer der nachfolgenden aufgeführten Verbindungen hergestellt werden, wobei diese auch in Form pharmazeutisch verwendbarer Salze, z. B. im Falle von Carbonsäuren, Salzen mit Base, wie Natriumsalze verwendet werden können:

5-Butyl-6-methyl-benzimidazol-2-carbonsäureäthylester,
5(6)-Butyl-benzimidazol-2-carbonsäureäthylester,
2-Äthoxymethyl-5-butyl-1-methyl-benzimidazol,
2-Äthoxymethyl-5-butyl-1,6-dimethyl-benzimidazol,
2-Äthoxymethyl-5-butyl-benzimidazol bzw.
5-Butyl-1-methyl-benzimidazol-2-carbonsäureäthylester.

Beispiel 13

In analoger Weise wie in den Beispielen 11 und 12 beschrieben kann man entsprechende pharmazeutische Präparate mit
2-Hydroxymethyl-5-butyl-1,6-dimethyl-benzimidazol,
5(6)-Methyl-benzimidazol-2-carbonsäure,
5,6-Dimethyl-benzimidazol-2-carbonsäure,
5-Butyl-1,6-dimethyl-benzimidazol-2-carbonsäure,
2-Hydroxymethyl-5(6)-methyl-benzimidazol oder
5,6-Dimethyl-2-hydroxymethyl-benzimidazol,
gegebenenfalls in Salzform, z. B. als Natriumsalz, als Wirkstoff herstellen.

**Patentansprüche**

1. Neue Benzimidazol-2-derivate der Formel

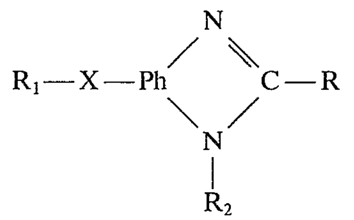

(I)

worin R eine gegebenenfalls veresterte oder amidierte Carboxygruppe oder eine gegebenenfalls verätherte oder veresterte Hydroxymethylgruppe ist, $R_1$ einen aliphatischen, cycloaliphatischen, aromatischen, araliphatischen, heterocyclischen oder heterocyclisch-aliphatischen Rest darstellt, $R_2$ für Wasserstoff oder einen aliphatischen Rest steht, und Ph eine den Rest $R_1-X$ enthaltende 1,2-Phenylengruppe darstellt, X Niederalkyliden oder eine direkte Bindung bedeutet, mit der Maßgabe, daß R von Alkoxymethyl oder $R_1-X-$ von Niederalkyl verschieden ist, wenn $R_2$ Wasserstoff, Alkyl oder Alkenyl bedeutet, daß weiterhin R von unsubstituiertem Carbamyl oder $R_1-X-$ von Alkyl verschieden ist, wenn $R_2$ Wasserstoff oder Alkyl bedeutet, und daß schließlich $R_1-X-Ph-$ von in 4- und/oder 5-Stellung durch Methyl substituiertem 1,2-Phenylen verschieden ist, wenn R Carboxy oder Hydroxymethyl darstellt und $R_2$ Wasserstoff bedeutet, und pharmazeutisch verwendbare Salze der genannten Verbindungen mit salzbildenden Eigenschaften.

2. Verbindungen gemäß Anspruch 1 der Formel I, worin R für freies Carboxy oder Hydroxymethyl, als verätherte Hydroxygruppe Niederalkyl, Hydroxyniederalkoxy, Niederalkoxyniederalkoxy oder Diniederalkylaminoniederalkoxy aufweisendes verestertes Carboxy oder veräthertes Hydroxymethyl, als Aminogruppe Amino, Hydroxyamino, Niederalkylamino, Diniederalkylamino oder Niederalkylenamino aufweisendes amidiertes Carboxy oder als veresterte Hydroxygruppe Niederalkanoyloxy oder gegebenenfalls durch Niederalkyl, Niederalkoxy und/oder Halogen substituiertes Benzoyloxy aufweisendes verestertes Hydroxymethyl steht, $R_1$ gegebenenfalls durch Niederalkoxy, Niederalkylthio, Niederalkylsulfinyl, Niederalkylsulfonyl, Phenylthio, Phenylsulfinyl oder Phenylsulfonyl substituiertes Niederalkyl, Niederalkenyl, Cycloalkyl, gegebenenfalls im Phenylteil durch Niederalkyl, Niederalkoxy oder Halogen substituiertes Phenyl oder Phenylniederalkyl, Furyl, Thienyl oder Pyridyl, oder Furylniederalkyl, Thienylniederalkyl oder Pyridylniederalkyl bedeutet, X Methylen bedeutet, $R_2$ Wasserstoff oder Niederalkyl darstellt, und Ph für den Rest $R_1-X-$ enthaltende und gegebenenfalls durch Niederalkyl, Niederalkoxy, Hydroxy und/oder Halogen substituiertes 1,2-Phenylen steht, und ihre pharmazeutisch verwendbaren Salze.

3. Verbindungen gemäß Anspruch 1 der Formel I, worin R für freies Carboxy, als verätherte

Hydroxygruppe Niederalkoxy oder Hydroxyniederalkoxy mit bis und mit 4 Kohlenstoffatomen aufweisendes verestertes Carboxy oder als Aminogruppe Amino oder Hydroxyamino, Niederalkylamino oder Diniederalkylamino, worin Niederalkyl bis und mit 4 Kohlenstoffatome enthält, aufweisendes amidiertes Carboxy steht oder Hydroxymethyl, als verätherte Hydroxygruppe Niederalkoxy mit bis zu 4 Kohlenstoffatomen oder Diniederalkylaminoniederalkoxy mit jeweils bis zu 4 Kohlenstoffatomen im Alkyl- bzw. Alkoxyteil aufweisendes veräthertes Hydroxymethyl oder als veresterte Hydroxygruppe Niederalkanoyloxy mit bis zu 7 Kohlenstoffatomen oder gegebenenfalls durch Niederalkyl, z. B. Methyl, Niederalkoxy, z. B. Methoxy und/oder Halogen, z. B. Chlor substituiertes Benzyloxy aufweisendes verestertes Hydroxymethyl bedeutet, $R_1$ Niederalkyl mit bis und mit 7 Kohlenstoffatomen, Niederalkoxy-, Niederalkylthio-, Niederalkansulfinyl- oder Niederalkansulfonyl-niederalkyl, worin die einzelnen Niederalkylreste bis und mit 4 Kohlenstoffatomen enthalten, Phenylthio-, Benzolsulfonyl- oder Benzolsulfonyl-niederalkyl, worin der Niederalkylrest bis und mit 4 Kohlenstoffatome enthält, Niederalkenyl mit bis und mit 5 Kohlenstoffatomen, Cycloalkyl mit bis und mit 7 Kohlenstoffatomen, gegebenenfalls durch Niederalkyl mit bis und mit 4 Kohlenstoffatomen, Niederalkoxy mit bis und mit 4 Kohlenstoffatomen und/oder Halogen mit Atomnummer bis und mit 35, substituiertes Phenyl oder Phenylniederalkyl mit bis und mit 4 Kohlenstoffatomen im Niederalkylrest, Furyl, Thienyl oder Pyridyl oder Furyl-, Thienyl- oder Pyridyl-niederalkyl mit bis und mit 4 Kohlenstoffatomen im Niederalkylrest bedeutet, X Methylen bedeutet, $R_2$ Wasserstoff oder Niederalkyl mit bis und mit 4 Kohlenstoffatomen darstellt, und Ph das den Rest der Formel $R_1-X-$ enthaltende, gegebenenfalls durch Niederalkyl mit bis und mit 4 Kohlenstoffatomen, Niederalkoxy mit bis zu und mit 4 Kohlenstoffatomen, Hydroxy und/oder Halogen mit Atomnummer bis und mit 35 substituiertes 1,2-Phenylen darstellt, wobei der Rest der Formel $R_1-X-$ irgendeine zur Substitution geeignete Stellung einnimmt, und ihre pharmazeutisch verwendbaren Salze.

4. Neue Benzimidazol-2-derivate der Formel

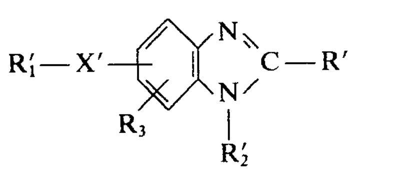

(Ia)

worin R′ für Carboxy oder für als verätherte Hydroxygruppe Niederalkoxy mit bis und mit 4 Kohlenstoffatomen aufweisendes verestertes Carboxy steht oder Hydroxymethyl oder als verätherte Hydroxymethylgruppe Niederalkoxy mit bis zu 4 Kohlenstoffatomen aufweisendes veräthertes Hydroxymethyl darstellt, und worin $R'_1$ Niederalkyl mit bis und mit 7 Kohlenstoffatomen, Cycloalkyl mit bis und mit 6 Ringkohlenstoffatomen oder Phenyl darstellt, X′ Methylen bedeutet, $R'_2$ für Wasserstoff oder Niederalkyl mit bis zu 4 Kohlenstoffatomen steht und $R_3$ Wasserstoff, Niederalkyl mit bis und mit 4 Kohlenstoffatomen, Niederalkoxy mit bis und mit 4 Kohlenstoffatomen oder Halogen mit Atomnummer bis und mit 35 bedeutet, mit der Maßgabe, daß R′ von als verätherte Hydroxygruppe Niederalkoxy mit bis und mit 4 Kohlenstoffatomen aufweisendem Niederalkoxymethyl verschieden ist, wenn $R'_1$ Niederalkyl mit bis und mit 6 Kohlenstoffatomen bedeutet, und pharmazeutisch verwendbare Salze von Verbindungen der Formel Ia mit salzbildenden Eigenschaften.

5. Verbindungen gemäß Anspruch 4 der Formel Ia, worin R′ Carboxy, Hydroxymethyl oder Niederalkoxycarbonyl oder Niederalkoxymethyl mit insgesamt bis zu 5 Kohlenstoffatomen bedeutet, $R'_1-X'$ Niederalkyl mit bis zu 8 Kohlenstoffatomen bedeutet und $R'_2$ und $R_3$ unabhängig voneinander Wasserstoff oder Niederalkyl mit bis zu 4 Kohlenstoffatomen darstellen, mit der Maßgabe, daß eine Methylgruppe $R'_1-X-$ nicht die 5(6)-Stellung des Benzimidazolringes einnimmt, wenn $R_3$ Wasserstoff oder in 6(5)-Stellung gebundenes Methyl ist, $R'_2$ Wasserstoff und R′ Carboxy oder Hydroxymethyl bedeutet, und pharmazeutisch verwendbare Salze der genannten Verbindungen mit salzbildenden Eigenschaften.

6. Verbindungen gemäß Anspruch 4 der Formel Ia, worin R′ für Carboxy oder für Niederalkoxycarbonyl mit insgesamt bis zu 5 Kohlenstoffatomen steht, worin $R'_1-X'-$ Niederalkyl mit bis und mit 7 Kohlenstoffatomen darstellt und $R'_2$ und $R_3$ Niederalkyl mit bis und mit 4 Kohlenstoffatomen darstellen, und pharmazeutisch verwendbare Salze der genannten Verbindungen, in denen R′ Carboxy ist, mit Basen.

7. Verbindungen gemäß Anspruch 4 der Formel Ia, worin R′ für Carboxy steht, $R'_1$ Niederalkyl mit bis zu 7 Kohlenstoffatomen darstellt, X′ Methylen bedeutet, $R'_2$ für Wasserstoff steht und $R_3$ Wasserstoff oder Niederalkyl mit bis zu 4 Kohlenstoffatomen darstellt, wobei der Rest $R'_1-X'-$ die 5- und ein Niederalkylrest $R_3$ die 6-Stellung des Benzimidazolringes einnimmt, und ihre Salze.

8. 5-Butyl-benzimidazol-2-carbonsäureäthylester, 5-Butyl-1,6-dimethyl-benzimidazol-2-carbonsäureäthylester, 5-Butyl-1-methyl-benzimidazol-2-carbonsäureäthylester, 5-Butyl-6-methyl-benzimidazol-2-carbonsäureäthylester, 5-Butyl-1,6-dimethyl-benzimidazol-2-carbonsäure und ihre pharmazeutisch verwendbaren Salze und 1,6-Dimethyl-5-butyl-2-hydroxymethyl-benzimidazol gemäß Anspruch 1.

9. Benzimidazol-2-derivate der Formel

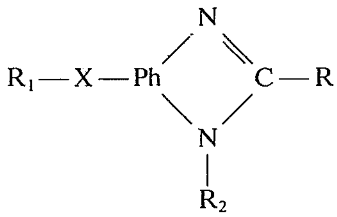

(I)

worin R eine gegebenenfalls veresterte oder amidierte Carboxygruppe oder eine gegebenenfalls verätherte oder veresterte Hydroxymethylgruppe ist, $R_1$ einen aliphatischen, cycloaliphatischen, aromatischen, araliphatischen, heterocyclischen oder heterocyclisch-aliphatischen Rest darstellt, $R_2$ für Wasserstoff oder einen aliphatischen Rest steht, und Ph eine den Rest $R_1-X$ enthaltende 1,2-Phenylengruppe darstellt, X Niederalkyliden oder eine direkte Bindung bedeutet, und pharmazeutisch verwendbare Salze der genannten Verbindungen mit salzbildenden Eigenschaften zur Anwendung in einem Verfahren zur prophylaktischen und/oder therapeutischen Behandlung des menschlichen oder tierischen Körpers.

10. Benzimidazol-2-derivate der Formel

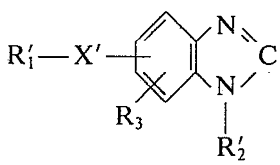

(Ia)

worin R' Carboxy, Hydroxymethyl oder Niederalkoxycarbonyl oder Niederalkoxymethyl mit insgesamt bis zu 5 Kohlenstoffatomen bedeutet, $R'_1-X'$ Niederalkyl mit bis zu 8 Kohlenstoffatomen bedeutet und $R'_2$ und $R_3$ unabhängig voneinander Wasserstoff oder Niederalkyl mit bis zu 4 Kohlenstoffatomen darstellen, und pharmazeutisch verwendbare Salze der genannten Verbindungen mit salzbildenden Eigenschaften zur Anwendung in einem Verfahren zur prophylaktischen und/oder therapeutischen Behandlung des menschlichen oder tierischen Körpers.

11. Verbindungen gemäß einem der Ansprüche 1–8 zur Anwendung in einem Verfahren zur prophylaktischen und/oder therapeutischen Behandlung des menschlichen oder tierischen Körpers.

12. 5-Methyl-benzimidazol-2-carbonsäure oder 5,6-Dimethylbenzimidazol-2-carbonsäure oder ein pharmazeutisch verwendbares Salz davon mit einer Base, 2-Äthoxymethyl-5-butyl-benzimidazol, 2-Äthoxymethyl-5-butyl-1-methyl-benzimidazol oder 2-Äthoxymethyl-5-butyl-1,6-dimethyl-benzimidazol zur Anwendung in einem Verfahren zur prophylaktischen und/oder therapeutischen Behandlung des menschlichen oder tierischen Körpers.

13. Pharmazeutische Präparate, enthaltend eine Verbindung gemäß einem der Ansprüche 1–10 und 12 neben üblichen pharmazeutischen Träger- und/oder Hilfsstoffen.

14. Verwendung von Verbindungen gemäß einem der Ansprüche 1–10 und 12 zur Herstellung eines Arzneimittels auf nicht-chemischem Wege.

15. Verfahren zur Herstellung neuer Benzimidazol-2-derivate der Formel

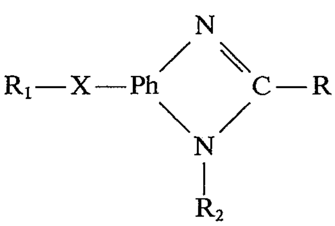

(I)

worin R eine gegebenenfalls veresterte oder amidierte Carboxygruppe oder eine gegebenenfalls verätherte oder veresterte Hydroxymethylgruppe ist, $R_1$ einen aliphatischen, cycloaliphatischen, aromatischen, araliphatischen, heterocyclischen oder heterocyclisch-aliphatischen Rest darstellt, $R_2$ für Wasserstoff oder einen aliphatischen Rest steht, und Ph eine den Rest $R_1-X$ enthaltende 1,2-Phenylengruppe darstellt, X Niederalkyliden oder eine direkte Bindung bedeutet, mit der Maßgabe, daß R von Alkoxymethyl oder $R_1X-$ von Niederalkyl verschieden ist, wenn $R_2$ Wasserstoff, Alkyl oder Alkenyl bedeutet, daß weiterhin R von unsubstituiertem Carbamyl oder $R_1-X-$ von Alkyl verschieden ist, wenn $R_2$ Wasserstoff oder Alkyl bedeutet, und daß schließlich $R_1-X-Ph$ von in 4- und/oder 5-Stellung durch Methyl substituiertem 1,2-Phenylen verschieden ist, wenn R Carboxy oder Hydroxymethyl darstellt und $R_2$ Wasserstoff bedeutet, und Salzen der genannten Verbindungen mit salzbildenden Eigenschaften, dadurch gekennzeichnet, daß man eine Verbindung der Formel II

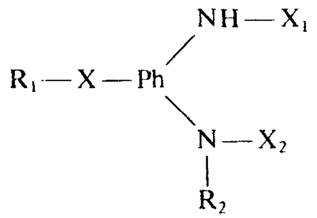

(II)

worin einer der Reste $X_1$ und $X_2$ eine Gruppe der Formel $-C(=O)-R$ und der andere Wasserstoff bedeutet, oder ein Salz davon cyclisiert oder in einer Verbindung der Formel

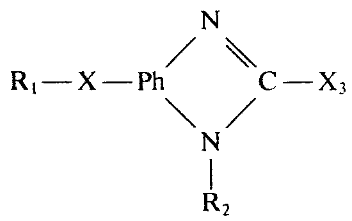

(III)

worin $X_3$ einen in die Gruppe R überführbaren Rest darstellt, $X_3$ in die Gruppe R überführt oder eine Verbindung der Formel

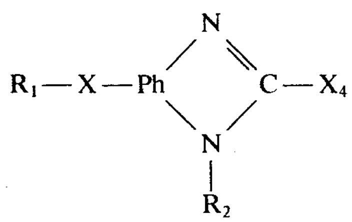

(IV)

mit einem reaktionsfähigen Derivat der Kohlensäure, z. B. mit Kohlendioxid oder einer Verbindung der Formel $R-X_5$ (V) umsetzt, worin $X_4$ ein Metallradikal und $X_5$ ein Halogenatom bedeutet, oder in einer Verbindung der Formel

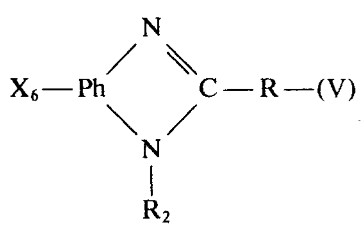

worin $X_6$ einen zu der Gruppe der Formel $R_1-X$ reduzierbaren Rest bedeutet, oder einem Salz davon die Gruppe $X_6$ zur gewünschten Gruppe der Formel $R_1-X-$ reduziert, und wenn erwünscht, eine so erhältliche Verbindung der Formel I in eine andere Verbindung der Formel I umwandelt, und/oder, wenn erwünscht, ein erhaltenes Salz in die freie Verbindung oder in ein anderes Salz und/oder eine freie salzbildende Verbindung in ein Salz überführt.

**Claims**

1. A novel benzimidazole-2-derivative of the formula

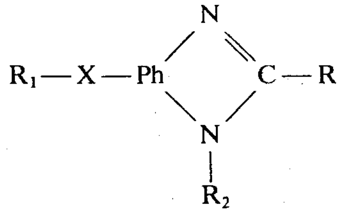

(I)

wherein R is a free, esterified or amidated carboxyl group or a free, etherified or esterified

hydroxymethyl group, $R_1$ is an aliphatic, cycloaliphatic, aromatic, araliphatic, heterocyclic or heterocyclic-aliphatic radical, $R_2$ is hydrogen or an aliphatic radical and Ph is a 1,2-phenylene group containing the radical $R_1-X$, and X is lower alkylidene or a direct bond, with the proviso that R is different from alkoxymethyl or $R_1-X-$ is different from lower alkyl if $R_2$ is hydrogen, alkyl or alkenyl, and that R is different from unsubstituted carbamyl or $R_1-X-$ is different from alkyl if $R_2$ is hydrogen or alkyl, and, finally, that $R_1-X-Ph$ is different from 1,2-phenylene substituted in the 4- and/or 5-position by methyl if R is carboxyl or hydroxymethyl and $R_2$ is hydrogen, or a pharmaceutically acceptable salt of such a compound with salt-forming properties.

2. A compound according to claim 1 of the formula I, wherein R is free carboxyl or hydroxymethyl, esterified carboxyl or etherified hydroxymethyl containing, as etherified hydroxyl group, lower alkoxy, hydroxy-lower alkoxy, lower alkoxy-lower alkoxy or di-lower alkylaminolower alkoxy, amidated carboxyl containing, as amino group, amino, hydroxylamino, lower alkylamino, di-lower alkylamino or lower alkyleneamino, or esterified hydroxymethyl containing, as esterified hydroxyl group, lower alkanoyloxy or benzoyloxy which is unsubstituted or substituted by lower alkyl, lower alkoxy and/or halogen, $R_1$ is lower alkyl which is unsubstituted or substituted by lower alkoxy, lower alkylthio, lower alkanesulfinyl, lower alkanesulfonyl, phenylthio, phenylsulfinyl or phenylsulfonyl, or lower alkenyl, cycloalkyl, phenyl or phenyl-lower alkyl which are unsubstituted or substituted in the phenyl moiety by lower alkyl, lower alkoxy or halogen, or furyl, thienyl or pyridyl, or furyl-lower alkyl, thienyl-lower alkyl or pyridyl-lower alkyl, X is methylene, $R_2$ is hydrogen or lower alkyl and Ph is 1,2-phenylene which contains the radical $R_1-X-$ and can be substituted by lower alkyl, lower alkoxy, hydroxyl and/or halogen, or a pharmaceutically acceptable salt thereof.

3. A compound according to claim 1 of the formula I, wherein R is free carboxyl, esterified carboxyl containing, as etherified hydroxyl group, lower alkoxy or hydroxy-lower alkoxy containing up to 4 carbon atoms inclusive, or amidated carboxyl containing, as the amino group, amino or hydroxyamino, lower alkylamino or di-lower alkylamino, in which lower alkyl contains up to 4 carbon atoms inclusive, or hydroxymethyl, etherified hydroxymethyl containing, as etherified hydroxyl group, lower alkoxy containing up to 4 carbon atoms inclusive or di-lower alkylamino-lower alkoxy containing up to 4 carbon atoms inclusive in each alkyl moiety and in the alkoxy moiety, or esterified hydroxymethyl containing, as esterified hydroxyl group, lower alkanoyloxy containing up to 7 carbon atoms inclusive or benzoyloxy which is unsubstituted or substituted by lower alkyl, for example methyl, lower alkoxy, for example methoxy, and/or halogen, for example chlorine, $R_1$ is lower alkyl containing up to 7 carbon atoms inclusive, lower alkoxy-, lower alkylthio-, lower alkanesulfinyl- or lower alkanesulfonyl-lower alkyl, in which the individual lower alkyl moieties contain up to 4 carbon atoms inclusive, phenylthio-lower alkyl, benzenesulfinyl-lower alkyl or benzenesulfonyl-lower alkyl, in which the lower alkyl moiety contains up to 4 carbon atoms inclusive, lower alkenyl containing up to 5 carbon atoms inclusive, cycloalkyl containing up to 7 carbon atoms inclusive, phenyl or phenyl-lower alkyl containing up to 4 carbon atoms inclusive in the lower alkyl moiety, each of which is unsubstituted or substituted by lower alkyl containing up to 4 carbon atoms inclusive, lower alkoxy containing up to 4 carbon atoms inclusive and/or halogen with an atomic number up to and including 35, or furyl, thienyl or pyridyl, or furyl-, thienyl- or pyridyl-lower alkyl containing up to 4 carbon atoms inclusive in the lower alkyl moiety, X is methylene, $R_2$ is hydrogen or lower alkyl containing up to 4 carbon atoms inclusive and Ph is 1,2-phenylene which contains the radical of the formula $R_1-X-$ and can be substituted by lower alkyl containing up to 4 carbon atoms inclusive, lower alkoxy containing up to 4 carbon atoms inclusive, hydroxyl and/or halogen with an atomic number up to and including 35, whilst the radical of the formula $R_1-X-$ is in any position suitable for substitution, or a pharmaceutically acceptable salt thereof.

4. A novel benzimidazole-2 derivative of the formula

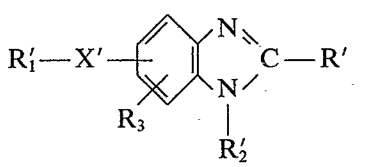

(Ia)

in which R′ is carboxyl, esterified carboxyl containing, as etherified hydroxyl group, lower alkoxy containing up to 4 carbon atoms inclusive, or hydroxymethyl or etherified hydroxymethyl containing, as etherified hydroxyl group, lower alkoxy containing up to 4 carbon atoms inclusive, and in which $R'_1$ is lower alkyl containing up to 7 carbon atoms inclusive, cycloalkyl containing up to 6 ring carbon atoms inclusive or phenyl, X′ is methylene, $R'_2$ is hydrogen or lower alkyl containing up to 4 carbon atoms inclusive and $R_3$ is hydrogen, lower alkyl containing up to 4 carbon atoms inclusive, lower alkoxy containing up to 4 carbon atoms inclusive or halogen with an atomic number up to and including 35, with the proviso that, if $R'_1$ is lower alkyl containing up to 6 carbon atoms inclusive, R′ is different from lower alkoxymethyl containing lower alkoxy of 1 to 4 carbon atoms as etherified hydroxyl group, or a pharmaceutically acceptable salt of a compound of the formula Ia with salt-forming properties.

5. A compound according to claim 4 of the formula Ia, wherein R′ is carboxyl, hydroxymethyl or lower

alkoxycarbonyl or lower alkoxymethyl or lower alkoxymethyl having a total of up to 5 carbon atoms inclusive, $R'_1-X'$ is lower alkyl containing up to 8 carbon atoms inclusive and each of $R'_2$ and $R_3$ independently is hydrogen or lower alkyl containing up to 4 carbon atoms inclusive, with the proviso that a methyl group $R'_1-X'-$ is not in the 5(6)-position of the benzimidazole ring when $R_3$ is hydrogen or methyl bonded in the 5(6)-position, $R'_2$ is hydrogen und R' is carboxyl or hydroxymethyl, or a pharmaceutically acceptable salt of such a compound with salt-forming properties.

6. A compound according to claim 4 of the formula Ia, wherein R' is carboxyl or lower alkoxycarbonyl containing a total of up to 5 carbon atoms inclusive and in which $R'_1-X'-$ is lower alkyl containing up to 7 carbon atoms inclusive and $R'_2$ and $R_3$ are lower alkyl containing up to 4 carbon atoms inclusive, or a pharmaceutically acceptabele salt of such a compound, in which R' is carboxyl, with a base.

7. A compound according to claim 4 of the formula Ia, wherein R' is carboxyl, $R'_1$ is lower alkyl containing up to 7 carbon atoms inclusive, X' is methylene, $R'_2$ is hydrogen and $R_3$ is hydrogen or lower alkyl containing up to 4 carbon atoms inclusive, whilst the radical $R'_1-X'-$ is in the 5-position of the benzimidazole ring and a lower alkyl radical $R_3$ is in the 6-position of the benzimidazole ring, or a salt thereof.

8. Ethyl 5-butyl-benzimidazole-2-carboxylate, ethyl 5-butyl-1,6-dimethylbenzimidazole-2-carboxylate, ethyl 5-butyl-1-methylbenzimidazole-2-carboxylate, ethyl 5-butyl-6-methylbenzimidazole-2-carboxylate, 5-butyl-1,6-dimethylbenzimidazole-2-carboxylic acid and pharmaceutically acceptable salts thereof, and 1,6-dimethyl-5-butyl-2-hydroxymethylbenzimidazole according to claim 1.

9. A benzimidazole-2 derivative of the formula

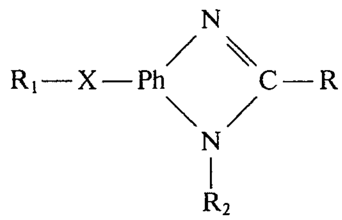

(I)

wherein R is a free, esterified or amidated carboxyl group or a free, etherified or esterified hydroxymethyl group, $R_1$ is an aliphatic, cycloaliphatic, aromatic, araliphatic, heterocyclic or heterocyclic-aliphatic radical, $R_2$ is hydrogen or an aliphatic radical and Ph is a 1,2-phenylene group containing the radical $R_1-X$, and X is lower alkylidene or a direct bond, or a pharmaceutically acceptable salt thereof for use in a prophylactic and/or therapeutic method of treating humans or animals.

10. A benzimidazole-2 derivative of the formula Ia

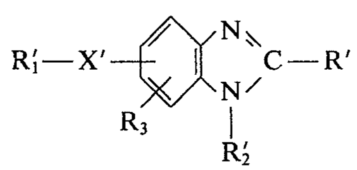

(Ia)

in which R' is carboxyl, hydroxymethyl or lower alkoxy-carbonyl or lower alkoxymethyl containing a total of up to 5 carbon atoms, $R'_1-X'$ is lower alkyl containing up to 8 carbon atoms inclusive, and each of $R'_2$ and $R_3$ independently is hydrogen or lower alkyl containing up to 4 carbon atoms inclusive, or a pharmaceutically acceptable salt thereof with salt-forming properties, for use in a prophylactic and/or therapeutic method of treating humans or animals.

11. A compound according to any one of claims 1 to 8 for use in a prophylactic and/or therapeutic method of treating humans or animals.

12. 5-Methyl-benzimidazole or 5,6-dimethylbenzimidazole-2-carboxylic acid or a pharmaceutically acceptable salt thereof with a base for use in a prophylactic and/or therapeutic method of treating humans or animals.

13. A pharmaceutical preparation containing a compound according to any one of claims 1 to 10 and 12, together with conventional pharmaceutical carriers and/or excipients.

14. A method of obtaining a medicament by a non-chemical route which comprises the use of a compound according to any one of claims 1 to 10 and 12.

15. A process for the production of a novel benzimidazole-2 derivative of the formula

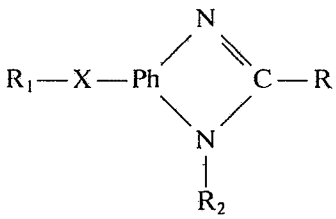

(I)

in which R is a free, esterified or amidated carboxyl group or a free, etherified or esterified hydroxymethyl group $R_1$ is an aliphatic, cycloaliphatic, aromatic, araliphatic, heterocyclic or heterocyclic-aliphatic radical, $R_2$ is hydrogen or an aliphatic radical and Ph is a 1,2-phenylene group containing the radical $R_1-X$, and X is lower alkylidene or a direct bond, with the proviso that R is different from alkoxymethyl or $R_1-X-$ is different from lower alkyl if $R_2$ is hydrogen, alkyl or alkenyl, and that R is different from unsubstituted carbamyl or $R_1-X-$ is different from alkyl if $R_2$ is hydrogen or alkyl, and, finally, that $R_1-X-Ph-$ is different from 1,2-phenylene substituted in the 4- and/or 5-position by methyl if R is carboxyl or hydroxymethyl and $R_2$ is hydrogen, or a pharmaceutically acceptable salt of such a compound with saltforming properties, characterised in that a compound of the formula II

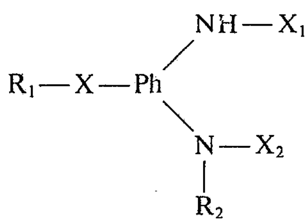

(II)

wherein one of the radicals $X_1$ and $X_2$ is a group of the formula $-C(=O)-R$ and the other is hydrogen, or a salt thereof, is cyclised, or, in a compound of the formula

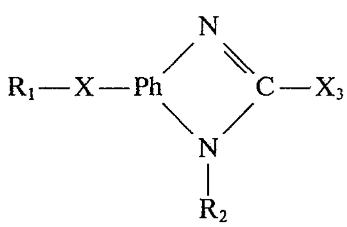

(III)

in which $X_3$ is a radical which is convertible to the group $R_3$, $X_3$ is so converted, or a compound of the formula

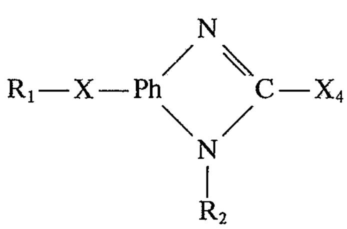

(IV)

is reacted with a reactive derivative of the carbonic acid, for example with carbon dioxide or a compound of the formula $R-X_5$ (V), in which formulae $X_4$ is a metal radical and $X_5$ is a halogen atom, or, in a compound of the formula

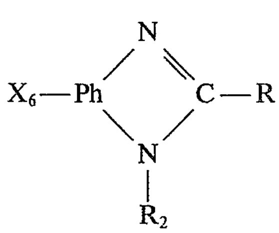

(V)

in which $X_6$ is a radical which is reducible to a group of the formula $R_1-X$, or in a salt thereof, $X_6$ is so reduced, and, if desired, a resultant compound of the formula I is converted into another compound of the formula I and/or, if desired, a resultant salt into the free compound or into another salt and/or a free salt-forming compound is converted into a salt.

**Revendications**

1. Nouveaux dérivés en position 2 de benzimidazoles, de formule

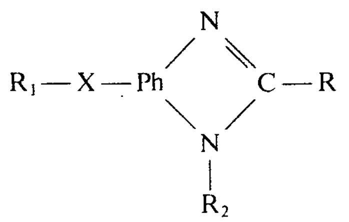

(I)

dans laquelle R représente un groupe carboxy éventuellement estérifié ou amidé ou un groupe hydroxyméthyle éventuellement éthérifié ou estérifié, $R_1$ représente un reste aliphatique, cycloaliphatique, aromatique, araliphatique, hétérocyclique ou hétérocyclique-aliphatique, $R_2$ représente l'hydrogène ou un reste aliphatique et Ph un groupe 1,2-phénylène portant le reste $R_1-X$, X représentant un groupe alkylidène inférieur ou une liaison directe, étant spécifié que R ne peut représenter un groupe alcoxyméthyle ou $R_1-X-$ un groupe alkyle inférieur lorsque $R_2$ représente l'hydrogène, un groupe alkyle ou alcényle, qu'en outre R ne peut représenter un groupe carbamyle non substitué ou $R_1-X-$ un groupe alkyle lorsque $R_2$ représente l'hydrogène ou un groupe alkyle, et que finalement $R_1-X-Ph-$ ne peut représenter un groupe 1,2-phénylène substitué par des groupes méthyles en position 4 et/ou 5 lorsque R représente un groupe carboxy ou hydroxyméthyle et $R_2$ l'hydrogène, et les sels acceptables pour l'usage pharmaceutique des composés en question capables de former des sels.

2. Composés selon la revendication 1, répondant à la formule I dans laquelle R représente un groupe carboxy ou hydroxyméthyle libre, un groupe carboxy estérifié ou hydroxyméthyle éthérifié contenant en tant que groupe hydroxy éthérifié un groupe alcoxy inférieur, hydroxyalcoxy inférieur, (alcoxy inférieur)-alcoxy inférieur ou di-(alkyle inférieur)-aminoalcoxy inférieur, un groupe carboxy amidé contenant en tant que groupe amino un groupe amino, hydroxyamino, alkylamino inférieur, di-(alkyle inférieur)-amino ou (alkylène inférieur)-amino ou un groupe hydroxyméthyle estérifié contenant en tant que groupe hydroxy estérifié un groupe alcanoyloxy inférieur ou un groupe benzoyloxy portant éventuellement des substituants alkyles inférieurs, alcoxy inférieurs et/ou halogéno, $R_1$ représente un groupe alkyle inférieur, alcényle inférieur, cycloalkyle portant éventuellement des substituants alcoxy inférieurs, alkylthio inférieurs, alkylsulfinyles inférieurs, alkylsulfonyles inférieurs, phénylthio, phénylsulfinyle ou phénylsulfonyle, un groupe phényle ou phényl-(alkyle inférieur) éventuellement substitué dans la partie phényle par des groupes alkyles inférieurs, alcoxy inférieurs ou des halogènes, un groupe furyle, thiényle ou pyridyle, ou un groupe furyl-(alkyle inférieur), thiényl-(alkyle inférieur) ou pyridyl-(alkyle inférieur), X représente le groupe méthylène, $R_2$ représente l'hydrogène ou un groupe alkyle inférieur et Ph représente le groupe 1,2-phénylène portant le reste $R_1-X-$ et éventuellement des substituants alkyles inférieurs, alcoxy inférieurs, hydroxy et/ou halogéno, et leurs sels acceptables pour l'usage pharmaceutique.

3. Composés selon la revendication 1, de formule I dans laquelle R représente le groupe carboxy libre, un groupe carboxy estérifié contenant en tant que groupe hydroxy éthérifié un groupe alcoxy inférieur ou hydroxyalcoxy inférieur contenant jusqu'à 4 atomes de carbone inclus ou un groupe carboxy amidé contenant en tant que groupe amino un groupe amino ou hydroxyamino, alkylamino inférieur ou di-(alkyle inférieur)-amino, les groupes alkyles inférieurs contenant jusqu'à 4 atomes de carbone inclus, ou un groupe hydroxyméthyle, ou un groupe hydroxyméthyle éthérifié contenant en tant que groupe hydroxy éthérifié un groupe alcoxy inférieur contenant jusqu'à 4 atomes de carbone ou di-(alkyle inférieur)-aminoalcoxy inférieur contenant jusqu'à 4 atomes de carbone dans les parties alkyles et alcoxy, ou un groupe hydroxyméthyle estérifié contenant en tant que groupe hydroxy estérifié un groupe alcanoyloxy inférieur contenant jusqu'à 7 atomes de carbone ou un groupe benzyloxy portant éventuellement des substituants alkyles inférieurs, par exemple méthyle, alcoxy inférieurs, par exemple méthoxy, et/ou halogéno, par exemple chloro, $R_1$ représente un groupe alkyle inférieur contenant jusqu'à 7 atomes de carbone inclus, (alcoxy inférieur)-, (alkylthio inférieur)-, (alcane inférieur)-sulfinyl- ou (alcane inférieur)-sulfonyl-alkyle inférieur, les groupes alkyles individuels contenant jusqu'à 4 atomes de carbone inclus, phénylthio-, benzène-sulfonyl- ou benzène-sulfonyl-alkyle inférieur, le groupe alkyle inférieur contenant jusqu'à 4 atomes de carbone inclus, alcényle inférieur contenant jusqu'à 5 atomes de carbone inclus, cycloalkyle contenant jusqu'à 7 atomes de carbone inclus, phényle ou phényl-(alkyle inférieur) contenant jusqu'à 4 atomes de

carbone inclus dans la partie alkyle inférieure et portant éventuellement des substituants alkyles inférieurs contenant jusqu'à 4 atomes de carbone inclus, alcoxy inférieurs contenant jusqu'à 4 atomes de carbone inclus et/ou halogéno de numéro atomique allant jusqu'à 35 inclus, un groupe furyle, thiényle ou pyridyle ou un groupe furyl-, thiényl- ou pyridyl-(alkyle inférieur) contenant jusqu'à 4 atomes de carbone inclus dans le reste alkyle inférieur, X représente le groupe méthylène, $R_2$ représente l'hydrogène ou un groupe alkyle inférieur contenant jusqu'à 4 atomes de carbone inclus et Ph représente le groupe 1,2-phénylène portant le reste de formule $R_1-X-$ et éventuellement des substituants alkyles inférieurs contenant jusqu'à 4 atomes de carbone inclus, alcoxy inférieurs contenant jusqu'à 4 atomes de carbone inclus, hydroxy et/ou halogéno de numéro atomique allant jusqu'à 35 inclus, le reste de formule $R_1-X-$ occupant une position quelconque appropriée à la substitution, et leurs sels acceptables pour l'usage pharmaceutique.

4. Nouveaux dérivés en position 2 de benzimidazoles de formule

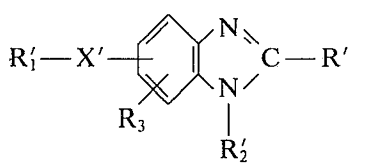

(Ia)

dans laquelle R' représente le groupe carboxy ou un groupe carboxy estérifié contenant en tant que groupe hydroxy éthérifié un groupe alcoxy inférieur contenant jusqu'à 4 atomes de carbone inclus, ou un groupe hydroxyméthyle, ou un groupe hydroxyméthyle éthérifié contenant en tant que groupe hydroxyméthyle éthérifié un groupe alcoxy inférieur contenant jusqu'à 4 atomes de carbone inclus, R' représente un groupe alkyle inférieur contenant jusqu'à 7 atomes de carbone inclus, cycloalkyle contenant jusqu'à 6 atomes de carbone cycliques ou phényle, X' représente le groupe méthylène, $R'_2$ représente l'hydrogène ou un groupe alkyle inférieur contenant jusqu'à 4 atomes de carbone et $R_3$ représente l'hydrogène, un groupe alkyle inférieur contenant jusqu'à 4 atomes de carbone inclus, alcoxy inférieur contenant jusqu'à 4 atomes de carbone inclus ou un halogène de numéro atomique allant jusqu'à 35 inclus, étant spécifié que R' ne peut représenter un groupe (alcoxy inférieur)-méthyle contenant en tant que groupe hydroxy éthérifié un groupe alcoxy inférieur contenant jusqu'à 4 atomes de carbone inclus lorsque $R'_1$ représente un groupe alkyle inférieur contenant jusqu'à 6 atomes de carbone inclus, et les sels acceptables pour l'usage pharmaceutique des composés de formule Ia capables de former des sels.

5. Composés selon la revendication 4, de formule Ia dans laquelle R' représente un groupe carboxy, hydroxyméthyle ou (alcoxy inférieur)-carbonyle ou (alcoxy inférieur)-méthyle contenant au total jusqu'à 5 atomes de carbone, $R'_1-X'$ représente un groupe alkyle inférieur contenant jusqu'à 8 atomes de carbone et $R'_2$ et $R_3$ représentent chacun, indépendamment l'un de l'autre, l'hydrogène ou un groupe alkyle inférieur contenant jusqu'à 4 atomes de carbone, étant spécifié qu'un groupe méthyle $R'_1-X-$ ne peut occuper la position 5(6) du cycle benzimidazole lorsque $R_3$ représente l'hydrogène ou un groupe méthyle fixé en position 6(5), $R'_2$ représente l'hydrogène et R' un groupe carboxy ou hydroxyméthyle, et les sels acceptables pour l'usage pharmaceutique des composés mentionnés capables de former des sels.

6. Composés selon la revendication 4, de formule Ia dans laquelle R' représente le groupe carboxy ou un groupe (alcoxy inférieur)-carbonyle contenant au total jusqu'à 5 atomes de carbone, $R'_1-X'-$ représente un groupe alkyle inférieur contenant jusqu'à 7 atomes de carbone inclus et $R'_2$ et $R_3$ représentent des groupes alkyles inférieurs contenant jusqu'à 4 atomes de carbone inclus, et les sels acceptables pour l'usage pharmaceutique des composés en question dans lesquels R' représente un groupe carboxy, et de bases.

7. Composés selon la revendication 4, de formule Ia dans laquelle R' représente le groupe carboxy, $R'_1$ un groupe alkyle inférieur contenant jusqu'à 7 atomes de carbone, X' le groupe méthylène, $R'_2$ l'hydrogène et $R_3$ l'hydrogène ou un groupe alkyle inférieur contenant jusqu'à 4 atomes de carbone, le reste $R'_1-X'-$ occupant la position 5 et un reste alkyle inférieur $R_3$ la position 6 du cycle benzimidazole, et leurs sels.

8. 5-butyl-benzimidazole-2-carboxylate d'éthyle, 5-butyl-1,6-diméthyl-benzimidazole-2-carboxylate d'éthyle, 5-butyl-1-méthyl-benzimidazole-2-carboxylate d'éthyle, 5-butyl-6-méthyl-benzimidazole-2-carboxylate d'éthyle, acide 5-butyl-1,6-diméthyl-benzimidazole-2-carboxylique et ses sels acceptables pour l'usage pharmaceutique, et 1,6-diméthyl-5-butyl-2-hydroxyméthyl-benzimidazole selon la revendication 1.

9. Dérivés en position 2 de benzimidazoles, de formule

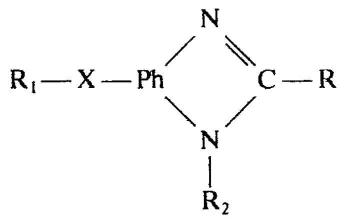

(I)

dans laquelle R représente un groupe carboxy éventuellement estérifié ou amidé ou un groupe hydroxyméthyle éventuellement éthérifié ou estérifié, $R_1$ représente un reste aliphatique, cycloaliphatique, aromatique, araliphatique, hétérocyclique ou hétérocyclique-aliphatique, $R_2$ représente l'hydrogène ou un reste aliphatique et Ph un groupe 1,2-phénylène portant le reste $R_1-X$, X représente un groupe alkylidène inférieur ou une liaison directe, et les sels acceptables pour l'usage pharmaceutique des composés mentionnés capables de former des sels pour l'utilisation dans un procédé pour le traitement prophylactique et/ou thérapeutique de l'organisme humain ou animal.

10. Dérivés en position 2 de benzimidazoles, de formule

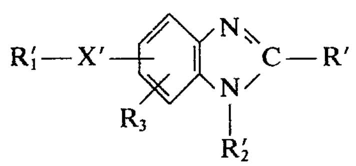

(Ia)

dans laquelle R' représente un groupe carboxy, hydroxyméthyle ou (alcoxy inférieur)-carbonyle ou (alcoxy inférieur)-méthyle contenant au total jusqu'à 5 atomes de carbone, $R'_1-X'$ représente un groupe alkyle inférieur contenant jusqu'à 8 atomes de carbone et $R'_2$ et $R_3$ représentent chacun, indépendamment l'un de l'autre, l'hydrogène ou un groupe alkyle inférieur contenant jusqu'à 4 atomes de carbone, et les sels acceptables pour l'usage pharmaceutique des composés mentionnés capables de former des sels pour l'utilisation dans un procédé pour le traitement prophylactique et/ou thérapeutique de l'organisme humain ou animal.

11. Composés selon l'une des revendications 1 à 8, pour l'utilisation dans un procédé pour le traitement prophylactique et/ou thérapeutique de l'organisme humain ou animal.

12. Acide 5-méthyl-benzimidazole-2-carboxylique ou acide 5,6-diméthyl-benzimidazole-2-carboxylique ou un sel acceptable pour l'usage pharmaceutique de l'un de ces acides et d'une base, 2-éthoxyméthyl-5-butyl-benzimidazole, 2-éthoxyméthyl-5-butyl-1-méthyl-benzimidazole ou 2-éthoxyméthyl-5-butyl-1,6-diméthyl-benzimidazole pour l'utilisation dans un procédé pour le traitement prophylactique et/ou thérapeutique de l'organisme humain ou animal.

13. Compositions pharmaceutiques contenant un composé selon l'une des revendications 1 à 10 et 12 avec des véhicules et/ou produits auxiliaires pharmaceutiques usuels.

14. Utilisation des composés selon l'une des revendications 1 à 10 et 12 pour la préparation d'un médicament par voie non chimique.

15. Procédé de préparation de nouveaux dérivés en position 2 de benzimidazoles, de formule

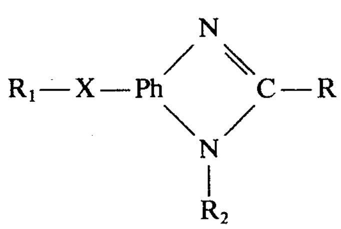

(I)

dans laquelle R représente un groupe carboxy éventuellement estérifié ou amidé ou un groupe hydroxyméthyle éventuellement éthérifié ou estérifié, $R_1$ représente un reste aliphatique, cycloaliphatique, aromatique, araliphatique, hétérocyclique ou hétérocyclique-aliphatique, $R_2$ représente l'hydrogène ou un reste aliphatique et Ph représente un groupe 1,2-phénylène portant le reste $R_1-X$, X représente un groupe alkylidène inférieur ou une liaison directe, étant spécifié que R ne peut représenter un groupe alcoxyméthyle ou $R_1-X-$ un groupe alkyle inférieur lorsque $R_2$ représente l'hydrogène, un groupe alkyle ou alcényle, qu'en outre R ne peut représenter un groupe carbamyle non substitué ou $R_1-X-$ un groupe alkyle lorsque $R_2$ représente l'hydrogène ou un groupe alkyle, et que finalement $R_1-X-Ph$ ne peut représenter un groupe 1,2-phénylène substitué par des groupes méthyles en positions 4 et/ou 5 lorsque R représente un groupe carboxy ou hydroxyméthyle et $R_2$ l'hydrogène, et des sels des composés mentionnés capables de former des sels, caractérisé en ce que l'on cyclise un composé de formule II

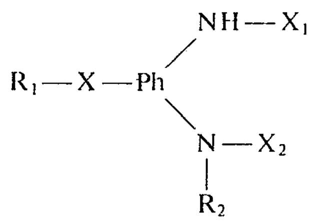

(II)

dans laquelle l'un des symboles $X_1$ et $X_2$ représente un groupe de formule $-C(=O)-R$ et l'autre l'hydrogène, ou un sel d'un tel composé, ou bien, dans un composé répondant à la formule

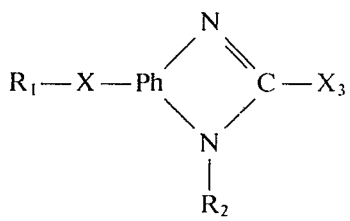

(III)

dans laquelle $X_3$ represente un reste convertible en le groupe R, on convertit $X_3$ en le groupe R, ou bien on fait réagir un composé de formule

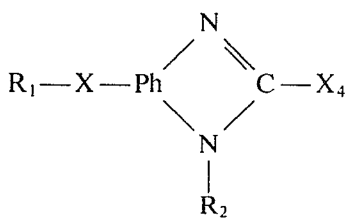

(IV)

avec un dérivé réactif de l'acide carbonique, par exemple l'anhydride carbonique, ou avec un composé de formule $R-X_5$ (V), $X_4$ représentant un radical métallique et $X_5$ un atome d'halogène, ou bien, dans un composé de formule

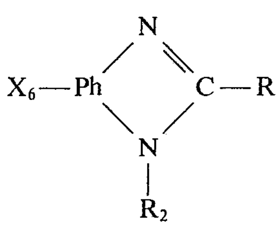

(V)

**dans laquelle $X_6$ représente un reste réductible en le groupe de formule $R_1-X$, ou dans un sel d'un tel composé, on réduit le groupe $X_6$ en le groupe de formule $R_1-X-$ recherché et si on le désire, on convertit un composé de formule I ainsi obtenu en un autre composé de formule I et/ou si on le désire, on convertit un sel obtenu en le composé libre ou en un autre sel et/ou on convertit un composé libre capable de former des sels en un sel.**